# EUROPEAN PATENT SPECIFICATION

(11) **EP 0 781 278 B1**
(45) Date of publication and mention of the grant of the patent: **14.03.2001**
(21) Application number: 95930289.4
(22) Date of filing: 28.08.1995
(51) Int. Cl.: C07D 337/08, A61K 31/38

(54) **NOVEL BENZOTHIEPINES HAVING ACTIVITY AS INHIBITORS OF ILEAL BILE ACID TRANSPORT AND TAUROCHOLATE UPTAKE**
BENZOTHEPINES MIT WIRKUNG ALS INHIBITOREN DES GALLENSÄURETRANSPORTS UND DER TAUROCHOLATE-AUFNAHME
NOUVELLES BENZOTHIEPINES A ACTIVITE INHIBITRICE DU TRANSPORT DES ACIDES BILIAIRES EN CAS D'ILEAS ET DE L'ABSORPTION DU TAUROCHOLATE

(30) Priority: 13.09.1994 US 305526
(43) Date of publication of application: 02.07.1997
(73) Proprietor: MONSANTO COMPANY, St. Louis, Missouri 63167 (US)
(72) Inventor: LEE, Len, Fang, St. Charles, MO 63303 (US); MILLER, Raymond, Eugene, Fairview Heights, IL 62208 (US); TREMONT, Samuel, Joseph, St. Louis, MO 63011 (US)
(74) Representative: Beil, Hans Christoph, Dr.
(86) International application number: US9510863
(87) International publication number: WO9608484

(56) References cited:
- EP-A- 0 350 846
- EP-A- 0 508 425
- WO-A-93/16055
- FR-A- 2 661 676
- FR-A- 2 698 873
- GB-A- 1 211 258
- US-A- 3 287 370
- TETRAHEDRON LETT. (TELEAY,00404039);89; VOL.30 (32); PP.4279-82, JADAVPUR UNIV.;DEP. CHEM.; CALCUTTA; 700032; INDIA (IN), PATRA R ET AL 'Conformational and steric requirements of the side chain for sulfur participation in benzothiepin derivatives'
- SYNTHESIS (SYNTBF,00397881);87; (9); PP.827-9, OSAKA MUNIC. TECH. RES. INST.;OSAKA; 536; JAPAN (JP), ISHINO Y ET AL 'Novel synthesis of 4,5-bis(arylthio)-2,3,4,5-tetrahydro-1-ben zothie pins: noteworthy cyclization by the reaction of 2-butynediol with arenethiols in the presence of zinc iodide'
- COLLECT. CZECH. CHEM. COMMUN. (CCCCAK);72; VOL.37 (12); PP.3808-16, RES. INST. PHARM. BIOCHEM.;PRAGUE; CZECH., KVIS F ET AL 'Benzocycloheptenes and heterocyclic analogs as potential drugs. VII. 4-Phenyl-2,3,4,5-tetrahydro-1-benzothiepin s and some related compounds'
- COLLECT. CZECH. CHEM. COMMUN. (CCCCAK);72; VOL.37 (4); PP.1195-1206, RES. INST. PHARM. BIOCHEM.;PRAGUE; CZECH., SINDELAR K ET AL 'Benzocycloheptenes and heterocyclic analogs as potential drugs. III. Further synthetic experiments in the series of 1-benzothiepin derivatives'
- J. CHEM. SOC. C (JSOOAX);71; (12); PP.2252-60, PARKE, DAVIS AND CO.;CHEM. RES. DEP.; HOUNSLOW/MIDDLESEX; ENGL., LOCKHART I M ET AL '4-Amino-2,3,4,5-tetrahydro-1-benzoxepin-5 -ols, 4-amino-2,3,4,5-tetrahydro-1-benzothiepin- 5-ols, and related compounds'
- COLLECT. CZECH. CHEM. COMMUN. (CCCCAK);68; VOL.33 (12); PP.4315-27, PHARM. RES. INST.;PRAGUE; CZECH., SINDELAR K ET AL 'Neurotropic and psychotropic agents. XXIX. Derivatives of 2,3,4,5-tetrahydro-1-benzothiepin'

## Description

This application is a continuation in part of US application 08/305526 filed September 12, 1994, now pending.

### BACKGROUND OF THE INVENTION

This invention relates to novel benzothiepines, derivatives and analogs thereof, pharmaceutical compositions containing them and their use in medicine, particularly in the prophylaxis and treatment of hyperlipidemic conditions, such as is associated with atherosclerosis, or hypercholesterolemia, in mammals.

It is well-settled that hyperlipidemic conditions associated with elevated concentrations of total cholesterol and low-density lipoprotein cholesterol are major risk factors for coronary heart disease and particularly atherosclerosis. Interfering with the circulation of bile acids within the lumen of the intestinal tract is found to reduce the levels of serum cholesterol in a causal relationship. Epidemiological data has accumulated which indicates such reduction leads to an improvement in the disease state of atherosclerosis. Stedronsky, in "Interaction of bile acids and cholesterol with nonsystemic agents having hypocholesterolemic properties," Biochimica et Biophysica Acta, 1210 (1994) 255-287 discusses the biochemistry, physiology and known active agents surrounding bile acids and cholesterol.

Pathophysiologic alterations are shown to be consistent with interruption of the enterohepatic circulation of bile acids in humans by Heubi, J.E., et al. See "Primary Bile Acid Malabsorption: Defective in Vitro Ileal Active Bile Acid Transport", Gastroenterology,1982:83:804-11.

In fact, cholestyramine binds the bile acids in the intestinal tract, thereby interfering with their normal enterohepatic circulation (Reihnér, E. et al, in "Regulation of hepatic cholesterol metabolism in humans: stimulatory effects of cholestyramine on HMG-CoA reductase activity and low density lipoprotein receptor expression in gallstone patients", Journal of Lipid Research, Volume 31, 1990, 2219-2226 and Suckling el al, "Cholesterol Lowering and bile acid excretion in the hamster with cholestyramine treatment", Atherosclerosis, 89(1991) 183-190). This results in an increase in liver bile acid synthesis by the liver using cholesterol as well as an upregulation of the liver LDL receptors which enhances clearance of cholesterol and decreases serum LDL cholesterol levels.

In another approach to the reduction of recirculation of bile acids, the ileal bile acid transport system is a putative pharmaceutical target for the treatment of hypercholesterolemia based on an interruption of the enterohepatic circulation with specific transport inhibitors (Kramer, et al, "Intestinal Bile Acid Absorption" The Journal of Biological Chemistry, Vol. 268, No. 24, Issue of August 25, pp. 18035-18046, 1993).

In a series of patent applications, eg Canadian Patent Application Nos. 2,025,294; 2,078,588; 2,085,782; and 2,085,830; and EP Application Nos. 0 379 161; 0 549 967; 0 559 064; and 0 563 731, Hoechst Aktiengesellschaft discloses polymers of various naturally occurring constituents of the enterohepatic circulation system and their derivatives, including bile acid, which inhibit the physiological bile acid transport with the goal of reducing the LDL cholesterol level sufficiently to be effective as pharmaceuticals and, in particular for use as hypocholesterolemic agents.

In vitro bile acid uptake inhibition is disclosed to show hypolipidemic activity in The Wellcome Foundation Limited disclosure of the world patent application number WO 93/16055 for "Hypolipidemic Benzothiazepine Compounds"

Selected benzothiepines are disclosed in world patent application number W093/321146 for numerous uses including fatty acid metabolism and coronary vascular diseases.

Other selected benzothiepines are known for use as hypolipaemic and hypocholesterolaemic agents, especially for the treatment or prevention of atherosclerosis as disclosed by application Nos. EP 508425, FR 2661676, and WO 92/18462, each of which is limited by an amide bonded to the carbon adjacent the phenyl ring of the fused bicyclo benzothiepine ring.

The above references show continuing efforts to find safe, effective agents for the prophylaxis and treatment of hyperlipidemic diseases and their usefulness as hypocholesterolemic agents.

Additionally selected benzothiepines are disclosed for use in various disease states not within the present invention utility. These are EP 568 898A as abstracted by Derwent Abstract No. 93-351589; WO 89/1477/A as abstracted in Derwent Abstract No. 89-370688; U.S. 3,520,891 abstracted in Derwent 50701R-B; US 3,287,370, US 3,389,144; US 3,694,446 abstracted in Derwent Abstr. No. 65860T-B and WO 92/18462.

The references
(a) THL, 1989, 30/32, p. 4279-82
(b) Collect. Czech. Chem. Comm. 1968, 33 (12), p. 4315-29 and
(c) EP-A 0 350 846
(d) Collect. Czech. Chem. Comm. 1972, 37 (4), p. 1195-1206 and 1972, 37 (12), p. 3808-16
disclose benzothiepines having
(i) an unsubstituted sulphur hetero atom in the benzothiepine ring (n is 0);
(ii) 3-position hydrogen substituent and in the 5 position OH or heterocyclic, or unsubstituted phenyl or alkyl.

The J. Chem. Soc. 1971, (12), p. 2252-60 (e) additionally discloses benzothiepines having:
(i) a sulphur hetero atom in the benzothiepine ring that is present at the sulfone (n=2);
(ii) 3-position hydrogen substituence;
(iii) a 4-position amino substituent;
(iv) a 5-position hydroxy substituent.

Compounds of above references (b), (d) and (e) are assumed to be useful as neurotopic, psychotropic or cardiovascular agents. Compounds of (c) are said to be useful as antiinflammatory or immunological agents (e.g. rheumatoid or dermatoid or similar conditions).

Synthesis, 1987 (9), p. 827-9 describes benzothiepines with an unsubtituted sulphur atom in the ring and having (substituted) thiophenyl-groups at positions 5 and 6. No pharmacological activities of such compounds are mentioned.

US-A 3,287,370 describes tetrahydrobenzthiepines wherein the position 5 is substituted with amino or substituted amino groups.

The present invention furthers such efforts with novel benzothiepines, pharmaceutical compositions and methods of use therefor.

### SUMMARY OF THE INVENTION

The present invention is for a compound of the formula (I) 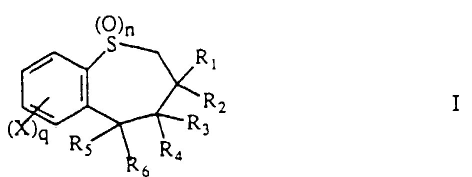 wherein
q is an integer of from 1 to
n is independently an integer of from 0 to 2,
R₁ and R₂ are independently H, C₁-C₁₀-alkyl or R₁ and R₂ taken together form C₃-C₁₀-cycloalkyl, preferably wherein both R₁ and R₂ cannot be hydrogen;
R₃ and R₄ are independently H, alkyl, aryl, OR, NRR', S(O)ₙR, or R₃ and R₄ together form =0, =NOH, =S, =NNRR', =NR", =CRR'where R, R'and R" are selected from H, alkyl, alkenylalkyl, alkynylalkyl, aryl, carboxyalkyl, carboalkoxyalkyl, cycloalkyl, or cyanalkyl; and provided that both R₃ and R₄ cannot be OH, NH₂ and SH;
R₅ is selected from alkyl, aryl, heterocycle, OR, NRR', S(O)ₙR wherein the alkyl, aryl, and heterocycle are each optionally substituted with alkyl, alkenyl, alkynyl, halogen, OR, NRR', S(O)ₙR, N0₂, haloalkyl, carboxy, carboalkoxy, CN, or N+RR'R"Y- wherein R, R' and R" are each independently as defined above, and Y is independently an anion, with the proviso that R₅ cannot be OH, NH₂, or NRR' when R₁, R₂, R₃, R₄, and R₆ are all hydrogen or R and R' are hydrogen or C₁-C₆-alkyl; with further proviso that R₅ cannot be OH or heterocyclic when R₁ and R₂ are hydrogen or alkyl and that R₅ cannot be C₁-C₆-alkyl or unsubsituted phenyl when R₁ and R₂ are both hydrogen and n is 0; and thatR₃, R₅ cannot both be thioaryl optionally substituted with C₁-C₆-alkyl, when R₄ is hydrogen and n is 0; and that
R₆ is selected from hydrogen or R₄ and R₆ together form -O-. or R₅ and R₆ together form a C₃-C₁₀-cycloalkylidine; with the proviso that R₄ and R₆ cannot together be -O- when R₃ is OH, NH₂ or SH;
X is selected from H, alkyl, alkenyl, alkynyl, halogen, OH, NH₂, OR, NRR', NROR', S(O)ₙR, N02, haloalkyl, carboxy, carboalkoxy, CN, or N+RR'R"Y- wherein R, R' and R" are each independently defined as above and Y is independently an anion;
wherein aryl means phenyl or naphthyl;
wherein heterocyclic is 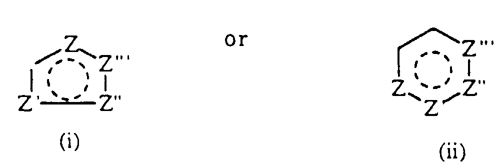 wherein Z, Z', Z" or Z"' is C, S, O, or N, with the proviso that one of Z, Z', Z" or Z"' is other than carbon, but is not O or S when attached to another Z atom by a double bond or when attched to another O or S atom, whereby optional substituents are attached to Z, Z', Z", Z"' only when each is C; or pharmaceutically acceptable salt or solvate thereof.

Preferred compounds include compounds of formula I wherein R₁ and R₂ cannot both be hydrogen;

Preferred compounds also include compounds of formula I wherein when R₅ is NRR', then R₃ or R₄ cannot be aryl.

The more preferred compounds are of the formula I wherein R₁ is butyl, R₂ is ethyl, R₃ is hydrogen, R₄ is hydroxy, R₅ is phenyl, q is 0, n is 2, and X is methoxy as shown below, or hydroxylamino or amino wherein each of R₂, R₄ and R₅ are in the same stereo relationship to the ring system which may be depicted as follows: 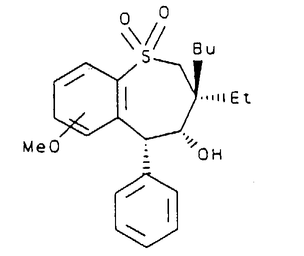 Other preferred compounds are those of formula I, wherein n is 2; or wherein R₅ is aryl optionally substituted with alkyl, alkenyl, alkynyl, halogen, OR, NRR', S(O)ₙR, NO₂, haloalkyl, carboxy, carboalkoxy, CN, or N+RR'R"Y-; or wherein R₃ and R₄ are independently H, or OH.

The present invention is also a pharmaceutical composition for the prophylaxis or treatment of a disease or condition for which a bile acid uptake inhibitor is indicated, such as hyperlipidemic condition, and in particular atherosclerosis, which comprises a compound of the formula I in an amount effective for inhibiting the bile acid uptake or the prophylaxis or treatment of the disease or condition benefitted thereby and a pharmaceutically acceptable carrier.

The present invention is also related to the use of a compound of the formula I in unit dosage form. for preparing a medicament for treating a disease or condition in humans for which a bile acid uptake inhibitor is indicated.

The present invention is also a process for the preparation of a compound of formula 1.

### DETAILED DESCRIPTION

"Alkyl", "alkenyl" and "alkynyl" unless otherwise noted are each of from one to six carbons for alkyl or two to six carbons for alkenyl and alkynyl in the present invention and, therefore, means methyl, ethyl, propyl, butyl, pentyl or hexyl and ethenyl, propenyl, butenyl, pentenyl, or hexenyl and ethynyl, propynyl, butynyl, pentynyl, or hexynyl respectively and isomers thereof. When each of these groups is refer-red to as a moiety in a parent molecule, such as alkenylalkyl, these definitions also apply.

"Aryl" is phenyl or naphthyl.

"Heterocyclo" is one of the following: 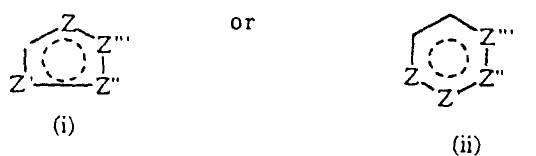 wherein Z, Z', Z" or Z"' is C, S, 0, or N, with the proviso that one of Z, Z', Z" or Z"' is other than carbon, but is not 0 or S when attached to another Z atom by a double bond or when attached to another 0 or S atom. Furthermore, the optional substituents are understood to be attached to Z, Z', Z" or Z"' only when each is C.

The halo group meant by "halogen" or meant in haloalkyl is a fluoro, chloro, bromo or iodo group.

Pharmaceutically acceptable salts are particularly suitable for medical applications because of their greater aqueous solubility relative to the parent. Such salts must clearly have a pharmaceutically acceptable anion or cation. Suitable pharmaceutically acceptable acid addition salts of the compounds of the present invention when possible include those derived from inorganic acids, such as hydrochloric, hydrobromic, phosphoric, metaphosphoric, nitric, sulphonic and sulphuric acids, and organic acids, such as acetic, benzenesulphonic, benzoic, citric, ethanesulphonic, fumaric, gluconic, glycollic, isothionic, lactic, lactobionic, maleic, malic, methansulphonic, succinic, -- toluenesulphonic, tartaric and trifluoroacetic acids. The chloride salt is particularly preferred for medical purposes. Suitable pharmaceutically acceptable base salts include ammonium salts, alkali metal salts, such as sodium and potassium salts, and alkaline earth salts, such as magnesium and calcium salts.

The anions of the definition of Y in the present invention are, of course, also required to be pharmaceutically acceptable and are also selected from the above list.

The compounds of the formula I may have at least two asymmetrical carbon atoms and therefore include rotational isomers. The invention includes all possible stereoisomers, in both pure form and in admixture, including racemic mixtures. Isomers can be prepared using conventional techniques, either by reacting enantiomeric starting materials or by separating isomers of a compound of formula I.

Isomers may include geometric isomers, e.g. when R₁ contains a double bond. All such isomers are contemplated for this invention.

In other words, diastereoisomers, enantiomers, racemates and tautomers are contemplated by the present invention.

The compounds of the formula I as referred to in the compositions and methods of use of the present invention are meant to include their salts, solvates and prodrugs as defined herein.

The term "a bile acid uptake inhibitor" as used in the present invention refers to inhibition of absorption of bile acids from the intestine of a mammal, such as a human, and includes increasing the fecal excretion of bile acids in a mammal, such as a human, as well as reducing the blood plasma or serum concentrations of cholesterol and cholesterol ester and more specifically reducing LDL and VLDL cholesterol in a mammal, such as a human. Conditions or diseases which benefit from the prophylaxis or treatment by bile acid uptake inhibition are, for example a hyperlipidemic condition, such as atherosclerosis.

The starting materials for use in the preparation of the compounds of the invention are known or can be prepared by conventional methods known to a skilled person or in an analogous manner to processes described in the art.

Generally, the compounds of the formula I can be prepared in one of the following procedures.

The compounds in this invention can be synthesized by the route shown in scheme 1.

Reaction of aldehyde II with formaldehyde and sodium hydroxide yields the hydroxyaldehyde III which is converted to mesylate IV with methansulfonyl chloride and triethylamine similar to the procedure described in Chem. Ber. **98**, 728-734 (1965). Reaction of mesylate IV with thiophenol V, prepared by the procedure described in WO 93/16055, in the presence of triethylamine yields keto-aldehyde VI which can be cyclized with the reagent, prepared from zinc and titanium trichloride in refluxing ethylene glycol dimethyl ether (DME), to give a mixture of 2,3-dihydrobenzothiepine VII and two racemic steroisomers of benzothiepin-(5*H*)-4-one VIII when R₁ and R₂ are nonequivalent. Oxidation of VII with 3 equivalents of m-chloroperbenzoic acid (MCPBA) gives isomeric sulfone-epoxides IX which upon hydrogenation with palladium on carbon as the catalyst yield a mixture of four racemic stereoisomers of 4-hydroxy-2,3,4,5-tetrahydrobenzothiepine-1,1-dioxides X and two racemic stereoisomers of 2,3,4,5-tetrahydro-benzothiepine-1,1-dioxides XI when R₁ and R₂ are nonequivalent.
Optically active compounds of this invention can be prepared by using optically active starting material III or by resolution of compounds X with optical resolution agents well known in the art as described in *J. Org. Chem.*, **39**, 3904 (19740, *ibid.,* **42**, 2781 (1977), and *ibid.,* **44**, 4891 (1979) 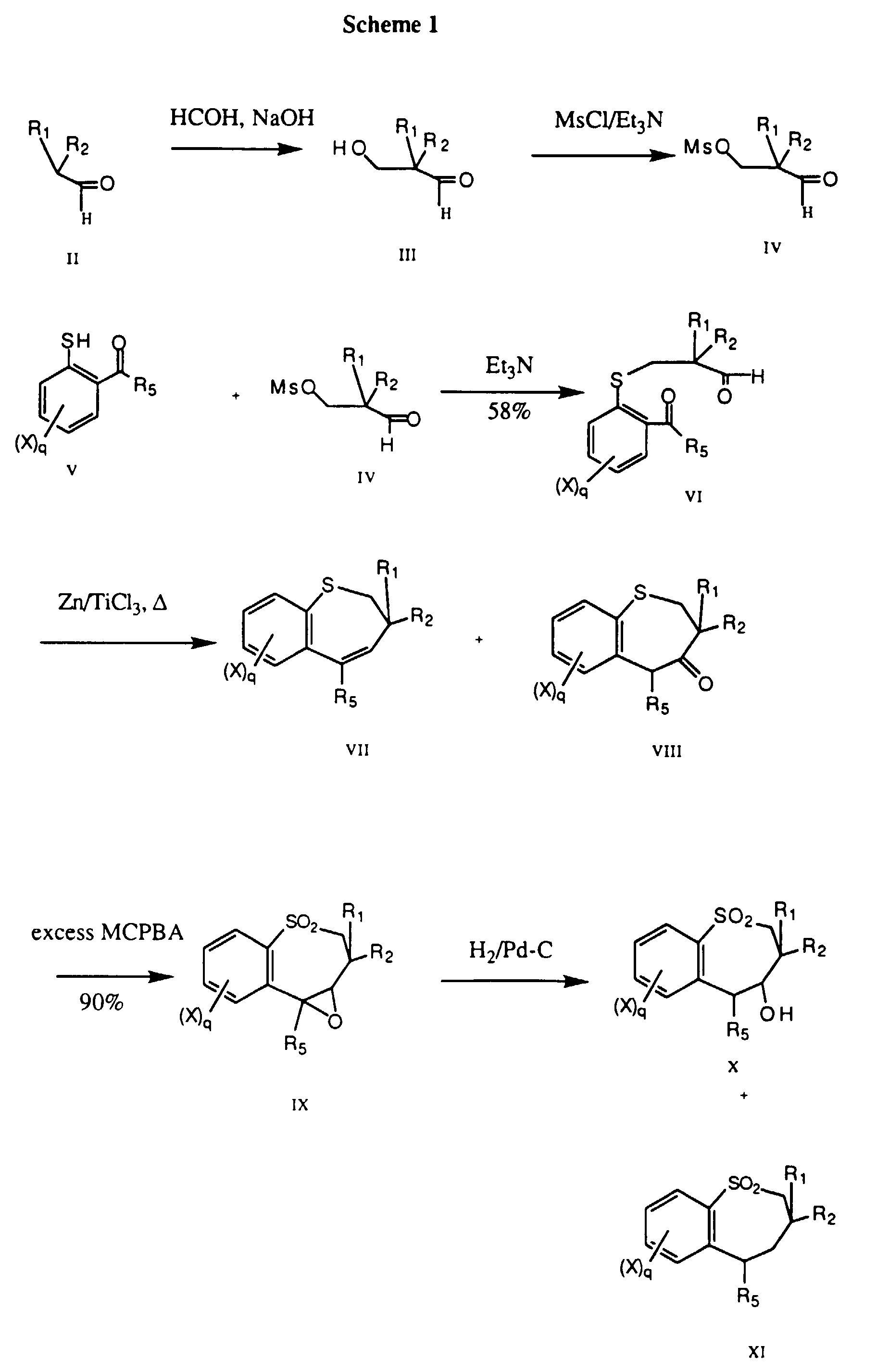 Alternatively, keto-aldehyde VI where R₂ is H can be prepared by reaction of thiophenol V with a 2-substituted acrolein. 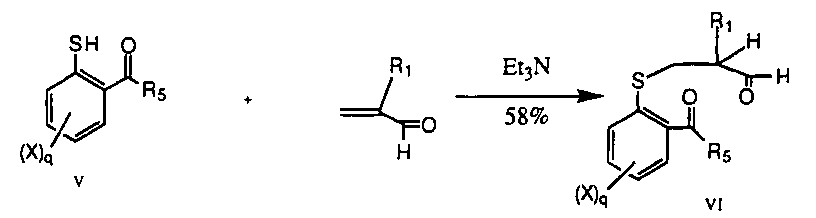 Benzothiepin-(5*H*)-4-one VIII can be oxidized with MCPBA to give the benzothiepin-(5*H*)-4-one-1,1-dioxide XII which can be reduced with sodium borohydride to give four racemic stereoisomers of X. The two stereoisomers of X, Xa and Xb, having the OH group and R₅ on the opposite sides of the benzothiepine ring can be converted to the other two isomers of X, Xc and Xd, having the OH group and R₅ on the same side of the benzothiepine ring by reaction in methylene chloride with 40-50% sodium hydroxide in the presence of a phase transfer catalyst (PTC). The transformation can also be carried out with potassium t-butoxide in THF. 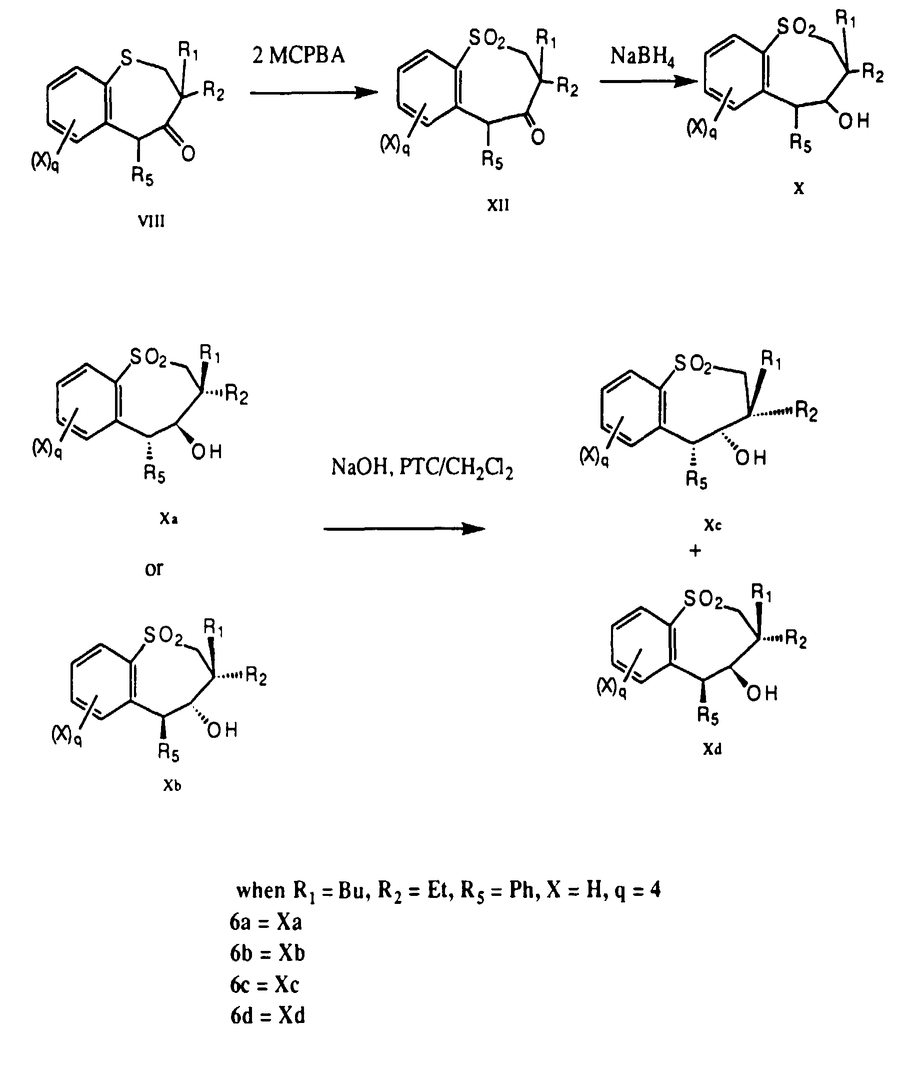 The compounds of this invention where R₅ is OR, NRR' and S(O)ₙR and R₄ is hydroxy can be prepared by reaction of epoxide IX where R₅ is H with thiol, alcohol, and amine in the presence of a base. 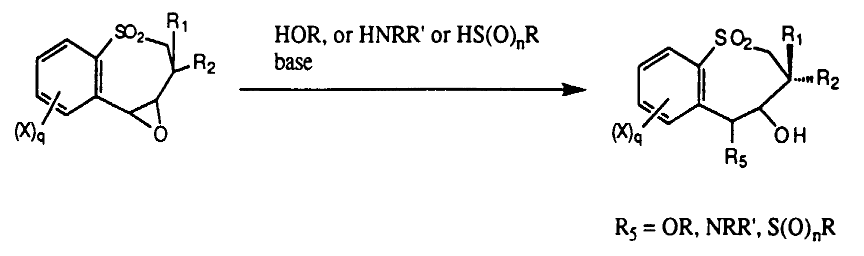 Another route to Xc and Xd of this invention is shown in scheme 2. Compound VI is oxidized to compound XIII with two equivalent of m-chloroperbenzoic acid. Hydrogenolysis of compound XIII with palladium on carbon yields compound XIV which can be cyclized with either potassium t-butoxide or sodium hydroxide under phase transfer conditions to a mixture of Xc and Xd. Separation of Xc and Xd can be accomplished with either HPLC or fractional crystallization. 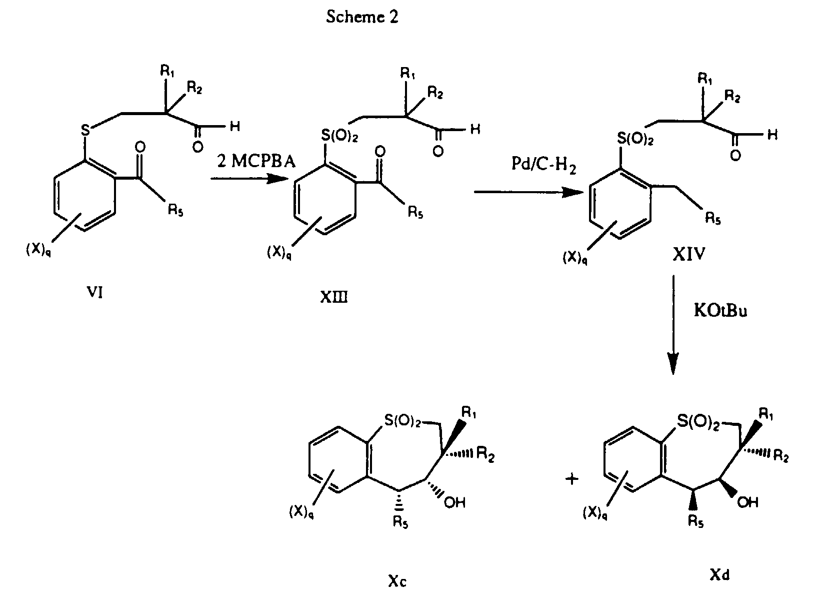 The thiopenols XVIII and V used in this invention can also be prepared according to the scheme 3. Alkylation of phenol XV with an arylmethyl chloride in nonpolar solvent according to the procedure in ***J. Chem. Soc.,* 2431-2432 (1958)** gives the ortho substituted phenol XVI. The phenol XVI can be converted to the thiophenol XVIII via the thiocarbamate XVII by the procedure described in ***J. Org. Chem.,* 31**, **3980 (1966)**. The phenol XVI is first reacted with dimethyl thiocarbamoyl chloride and triethylamine to give thiocarbamate XVII which is thermal rearranged at 200-300 °C and the rearranged product is hydrolyzed with sodium hydroxide to yield the thiophenol XVIII. Similarly, Thiophenol V can also be prepared from 2-acylphenol XIX via the intermediate thiocarbamate XX. 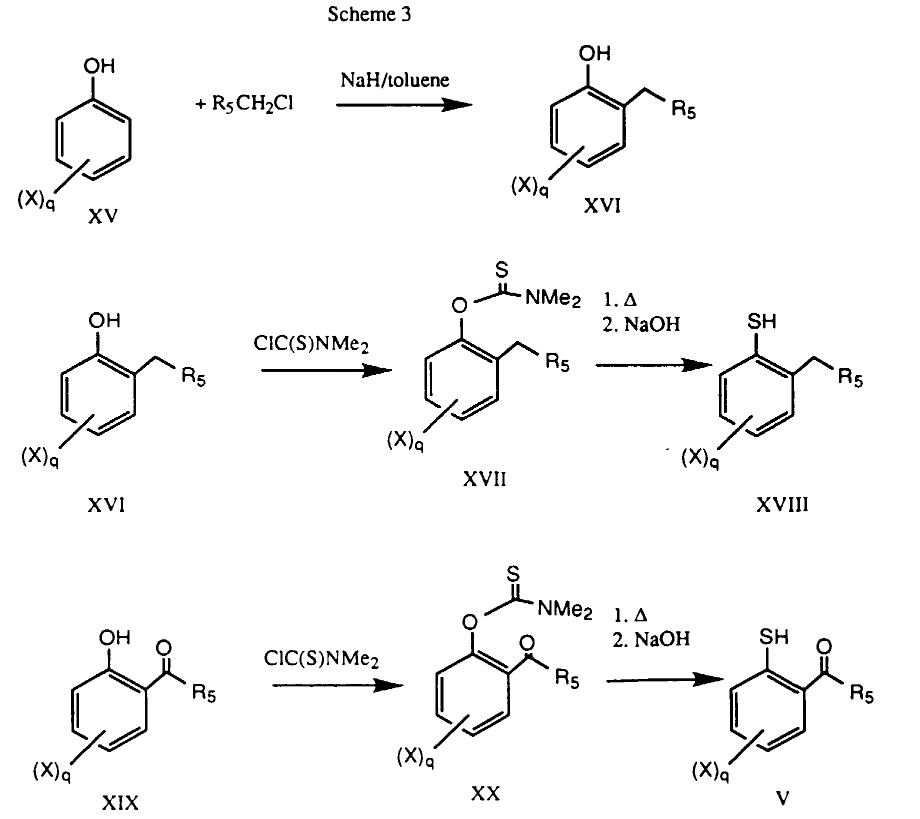 Scheme 4 shows another route to benzothiepine-1,1-dioxides Xc and Xd starting from the thiophenol XVIII. Compound XVIII can be reacted with mesylate IV to give the sulfide-aldehyde XXI. Oxidation of XXI with two equivalents of MCPBA yields the sulfone-aldehyde XIV which can be cyclized with potassium t-butoxide to a mixture of Xc and Xd. Cyclyzation of sulfide-aldehyde with potassium t-butoxide also gives a mixture of benzothiepine XXIIc and XXIId. 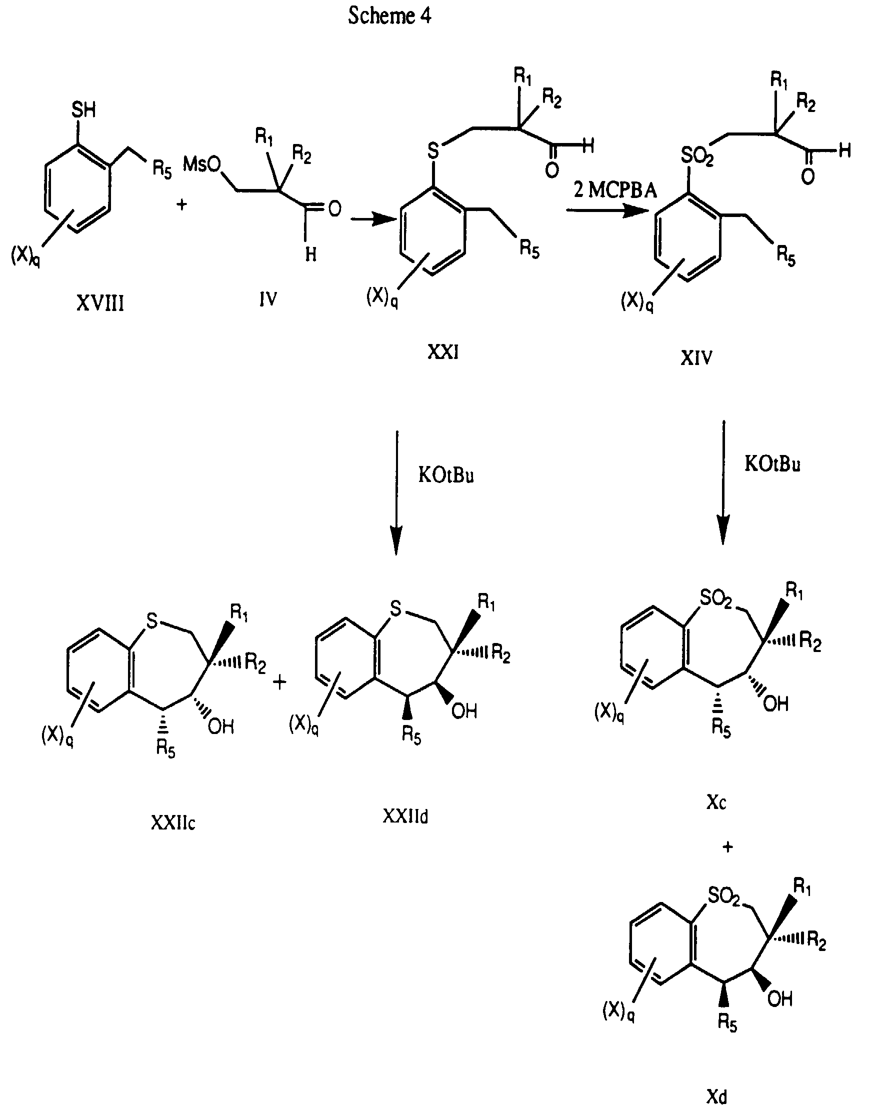 Examples of amine and hydroxylamine containing compounds of this invention can be prepared as shown in scheme 5 and scheme 6. 2-Chloro-4-nitrobenzophenone is reduced with triethylsilane and trifluoromethane sulfonic acid to 2-chloro-4-nitrodiphenylmethane **32**. Reaction of **32** with lithium sulfide followed by reacting the resulting sulfide with mesylate IV gives sulfide-aldehyde XXIII. Oxidation of XXIII with 2 equivalents of MCPBA yields sulfone-aldehyde XXIV which can be reduced by hydrogenation to the hydroxylamine XXV. Protecting the hydroxylamine XXV with di-t-butyldicarbonate gives the *N,O*-di-(t-butoxycarbonyl)hydroxylamino derivative XXVI. Cyclization of XXVI with potassium t-butoxide and removal of the t-butoxycarbonyl protecting group gives the a mixture of hydroxylamino derivative XXVIIc and XXVIId. The primary amine XXXIIIc and XXXIIId derivatives can also be prepared by further hydrogenation of XXIV or XXVIIc and XXVIId. 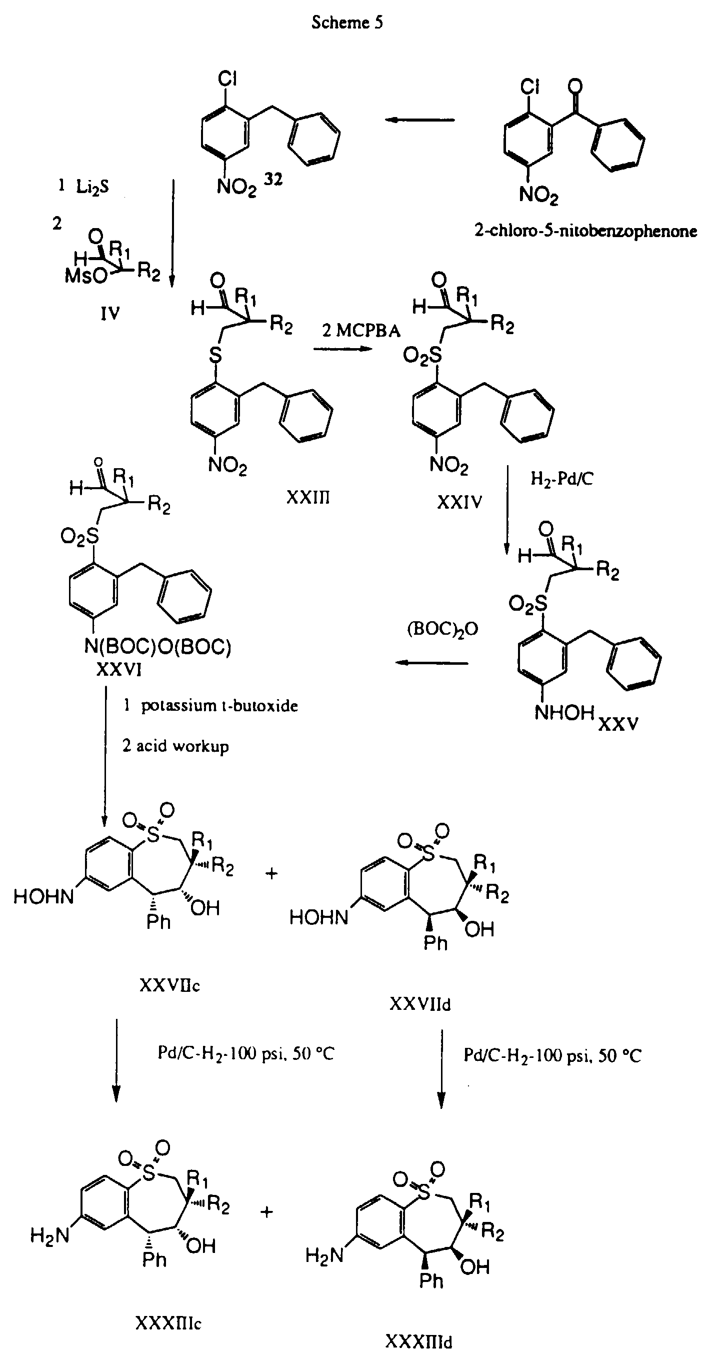 Reduction of the sulfone-aldehyde XXV with hydrogen followed by reductive alkylation of the resulting amino derivative with hydrogen and an aldehyde catalyzed by palladium on carbon in the same reaction vessel yields the substituted amine derivative XXVIII. Cyclization of XXVIII with potassium t-butoxide yields a mixture of substituted amino derivatives of this invention XXIXc and XXIXd. 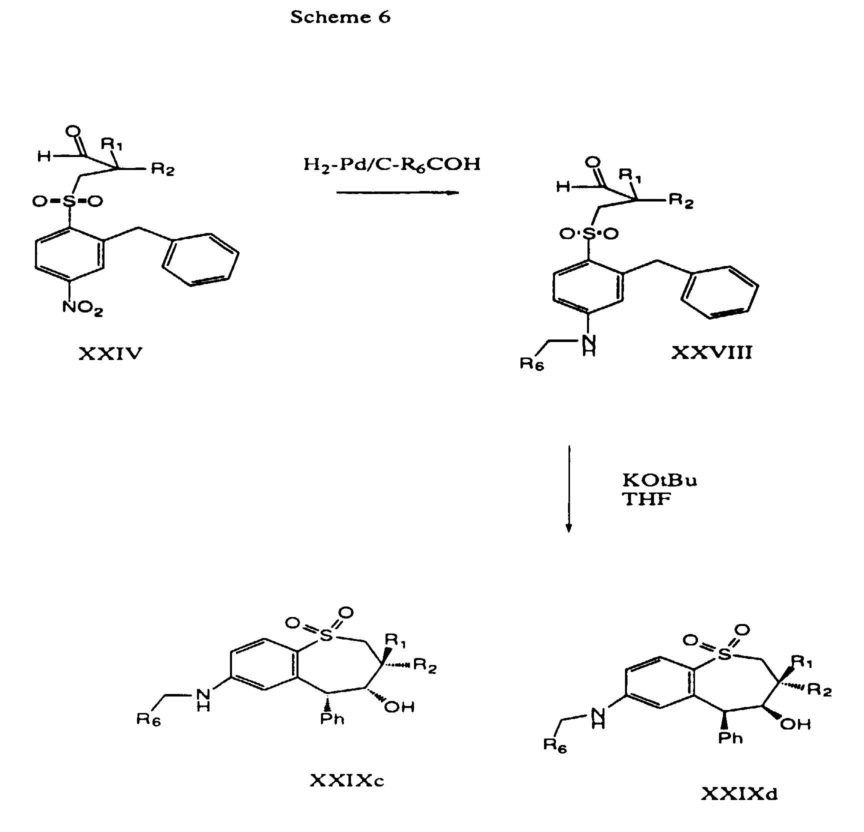 Scheme 7 describes one of the methods in introducing a substituent to the aryl ring at the 5-position of benzothiepine. Iodination of 5-phenyl derivative XXX with iodine catalyzed by mercuric triflate gives the iodo derivative XXXI which upon palladium catalyzed carbonylation in an alcohol yields the carboxylate XXXII. Hydrolysis of the carboxylate and derivatization of the resulting acid to acid derivatives are well known in the art. 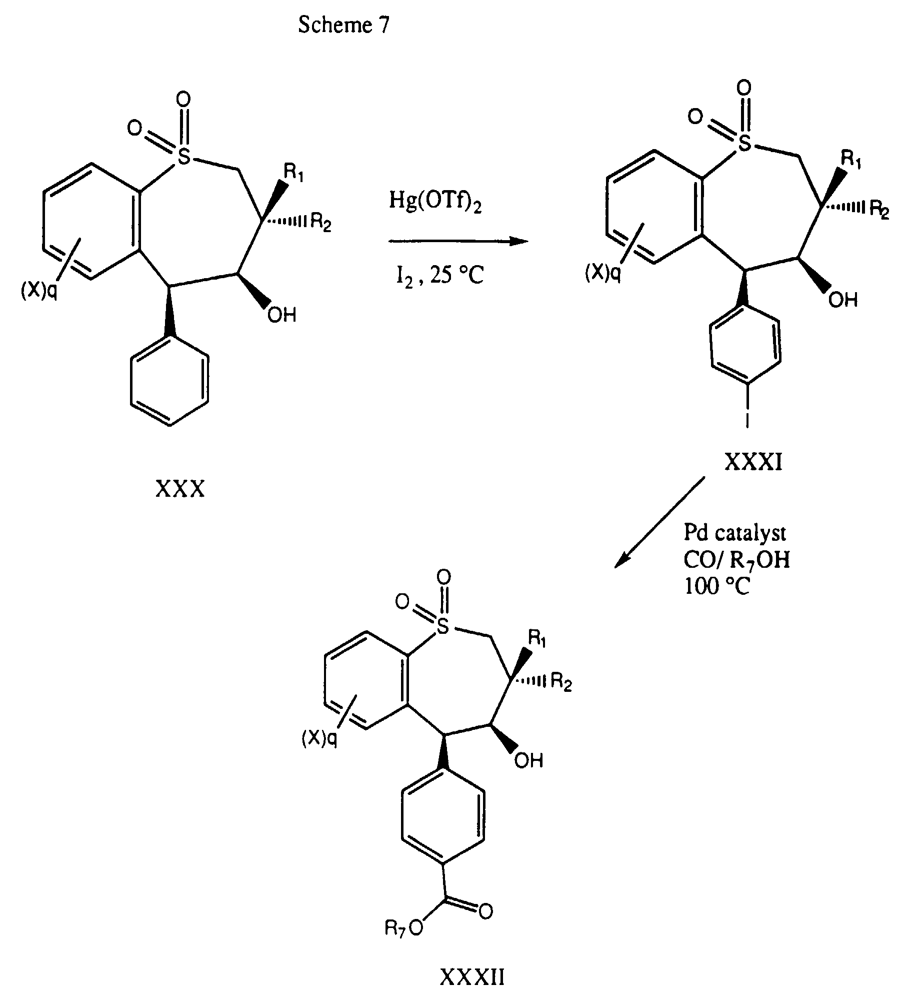 Abbreviations described in this invention are:
THF---tetrahydrofuran
PTC---phase transfer catalyst
Aliquart 336---methyltricaprylylammonium chloride
MCPBA---m-chloroperbenzoic acid
Celite--- a brand of diatomaceous earth filtering aid
DMF---dimethylformamide
DME----ethylene glycol dimethyl ether
BOC---t-butoxycarbonyl group

The following examples are not meant to be limiting.

### EXAMPLES

### Preparation 1

### 2-Ethyl-2-(mesyloxymethyl)hexanal (1)

To a cold (10 °C) solution of 12.6 g (0.11 mole) of methanesulfonyl chloride and 10.3 g (0.13 mole) of triethylamine was added dropwise 15.8 g of 2-ethyl-2-(hydroxymethyl)hexanal, prepared according to the procedure described in Chem. Ber. **98**, 728-734 (1965), while maintaining the reaction temperature below 30 °C. The reaction mixture was stirred at room temperature for 18 h, quenched with dilute HCl and extracted with methlyene chloride. The methylene chloride extract was dried over MgSO₄ and concentrated in vacuo to give 24.4 g of brown oil.

### Preparation 2

### 2-((2-Benzoylphenylthio)methyl)-2-ethylhexanal (2)

A mixture of 31 g (0.144 mol) of 2-mercaptobenzophenone, prepared according to the procedure described in WO 93/16055, 24.4 g (0.1 mole) of 2-ethyl-2-(mesyloxymethyl)-hexanal (**1**), 14.8 g (0.146 mole) of triethylamine, and 80 mL of 2-methoxyethyl ether was held at reflux for 24 h. The reaction mixture was poured into 3N HCl and extracted with 300 mL of methylene chloride. The methylene chloride layer was washed with 300 mL of 10% NaOH, dried over MgSO₄ and concentrated in vacuo to remove 2-methoxyethyl ether. The residue was purified by HPLC (10% EtOAc-hexane) to give 20.5 g (58%) of **2** as an oil.

### Example 1

### 3-Butyl-3-ethyl-5-phenyl-2,3-dihydrobenzothiepine (3), cis-3-Butyl-3-ethyl-5-phenyl-2,3-dihydrobenzothiepin-(5H)4-one (4a) and trans-3-Butyl-3-ethyl-5-phenyl-2,3-dihydro-benzothiepin-(5H)4-one (4b)

A mixture of 2.6 g (0.04 mole) of zinc dust, 7.2 g (0.047 mole) of TiCl₃ and 80 mL of anhydrous ethylene glycol dimethyl ether (DME) was held at reflux for 2 h. The reaction mixture was cooled to 5 °C. To the reaction mixture was added dropwise a solution of 3.54 g (0.01 mole) of **2** in 30 mL of DME in 40 min. The reaction mixture was stirred at room temperature for 16 h and then was held at reflux for 2 h and cooled before being poured into brine. The organic was extract into methylene chloride. The methylene chloride extract was dried over MgSO₄ and concentrated in vacuo. The residue was purified by HPLC (hexane) to give 1.7 g (43%) of **3** as an oil in the first fraction. The second fraction was discarded and the third fraction was further purified by HPLC (hexane) to give 0.07 g (2%) of **4a** in the earlier fraction and 0.1 g (3%) of **4b** in the later fraction.

### Example 2

### cis-3-Butyl-3-ethyl-5-phenyl-2,3-dihydrobenzothiepin-(5H)4-one-1,1-dioxide (5a) and trans-3-Butyl-3-ethyl-5-phenyl-2,3-dihydrobenzothiepin-(5H)4-one-1,1-dioxide (5b)

To a solution of 1.2 g (3.5 mmole) of 50-60% MCPBA in 20 mL of methylene chloride was added 0.59 g (1.75 mmole) of a mixture of **4a** and **4b** in 10 mL of methylene chloride. The reaction mixture was stirred for 20 h. An additional 1.2 g (1.75 mmole) of 50-60% MAPBA was added and the reaction mixture was stirred for an additional 3 h then was triturated with 50 mL of 10% NaOH. The insoluble solid was filtered. The methylene chloride layer of the filtrate was washed with brine, dried over MgSO₄, and concentrated in vacuo. The residual syrup was purified by HPLC (5% EtOAc-hexane) to give 0.2 g (30%)of **5a** as an oil in the first fraction and 0.17 g (26%) of **5b** as an oil in the second fraction.

### Example 3

### (3α,4α,5β) 3-Butyl-3-ethyl-4-hydroxy-5-phenyl-2,3,4,5-tetrahydrobenzothiepine-1,1-dioxide (6a), (3α,4β,5α) 3-Butyl-3-ethyl-4-hydroxy-5-phenyl-2,3,4,5-tetrahydro-benzothiepine-1,1-diode (6b), (3α,4α,5α) 3-Butyl-3-ethyl-4-hydroxy-5-phenyl-2,3,4,5-tetrahydrobenzothiepine-1,1-dioxide (6c), and (3α,4β,5β) 3-Butyl-3-ethyl-4-hydroxy-5-phenyl-2,3,4,5-tetrahydrobenzothiepine-1,1-dioxide (6d)

### A. Reduction of 5a and 5b with Sodium Borohydride

To a solution of 0.22 g (0.59 mmole) of **5b** in 10 mL of ethanol was added 0.24 g (6.4 mmole) of sodium borohydride. The reaction mixture was stirred at room temperature for 18 h and concentrated in vacuo to remove ethanol. The residue was triturated with water and extracted with methylene chloride. The methylene chloride extract was dried over MgSO₄ and concentrated in vacuo to give 0.2 g of syrup. In a separate experiment, 0.45 g of **5a** was treated with 0.44 g of sodium borohydride in 10 mL of ethanol and was worked up as described above to give 0.5 g of syrup which was identical to the 0.2 g of syrup obtained above. These two materials were combined and purified by HPLC using 10% EtOAc-hexane as eluant. The first fraction was 0.18 g (27%) of **6a** as a syrup. The second fraction was 0.2 g (30%) of **6b** also as a syrup. The column was then eluted with 20% EtOAc-hexane to give 0.077 g (11%) of **6c** in the third fraction as a solid. Recrystallization from hexane gave a solid, mp 179-181 °C. Finally, the column was eluted with 30% EtOAc-hexane to give 0.08 g (12%) of **6d** in the fourth fraction as a solid. Recrystallization from hexane gave a solid, mp 160-161 °C.

### B. Conversion of 6a to 6c and 6d with NaOH and PTC

To a solution of 0.29 g (0.78 mmole) of **6a** in 10 mL CH₂Cl₂, was added 9 g of 40% NaOH. The reaction mixture was stirred for 0.5 h at room temperature and was added one drop of Aliquat-336 (methyltricaprylylammonium chloride) phase transfer catalyst (PTC). The mixture was stirred for 0.5 h at room temperature before being treated with 25 mL of ice-crystals then was extracted with CH₂Cl₂ (3x10 ml), dried over MgSO₄ and concentrated in vacuo to recover 0.17 g of a colorless film. The components of this mixture were separated using an HPLC and eluted with EtOAc-hexane to give 12.8 mg (4%) of 2-(2-benzylphenylsulfonylmethyl)-2-ethylhexenal in the first fraction, 30.9 mg (11%) of **6c** in the second fraction and 90.0 mg (31%) of **6d** in the third fraction.

### Oxidation of 6a to 5b

To a solution of 0.20 g (0.52 mmole) of **6a** in 5 mL of CH₂Cl₂ was added 0.23 g (1.0 mmole) of pyridinium chlorochromate. The reaction mixture was stirred for 2 h then was treated with additional 0.23 g of pyridinium chlorochromate and stirred overnight. The dark reaction mixture was poured into a ceramic filterfrit containing silica gel and was eluted with CH₂Cl₂. The filtrate was concentrated in vacuo to recover 167 mg (87%) of **5b** as a colorless oil.

### Example 4

### 3-Butyl-3-ethyl-5-phenyl-2,3-dihydrobenzothiepine-1,1-diode (7)

To a solution of 5.13 g (15.9 mmole) of **3** in 50 mL of CH₂Cl₂ was added 10 g (31.9 mmole)of 50-60% MCPBA (m-chloroperoxybenzoic acid) portionwise causing a mild reflux and formation of a white solid. The reaction mixture was allowed to stir overnight under N₂ and was triturated with 25 mL of water followed by 50 mL of 10% NaOH solution. The organic was extracted into CH₂Cl₂ (4x20 mL). The CH₂Cl₂ extract was dried over MgSO₄ and evaporated to dryness to recover 4.9 g (87%) of an opaque viscous oil.

### Example 5

### (1aα,2β,8bα) 2-Butyl-2-ethyl-8b-phenyl-1a,2,3,8b-tetrahydrobenzothiepino[4,5-b]oxirene-4,4-dioxide (8a) (1aα,2α,8bα) 2-Butyl-2-ethyl-8b-phenyl-1a,2,3,8b-tetrahydrobenzothiepino[4,5-b]oxirene-4,4-diode (8b)

To 1.3 g (4.03 mole) of **3** in 25 mL of CHCl₃ was added portionwise 5 g (14.1 mmole) of 50-60 % MCPBA causing a mild exotherm. The reaction mixture was stirred under N₂ overnight and was then held at reflux for 3 h. The insoluble white slurry was filtered. The filtrate was extracted with 10% potassium carbonate (3x50 mL), once with brine, dried over MgSO₄, and concentrated in vacuo to give 1.37 g of a light yellow oil. Purification by HPLC gave 0.65 g of crystalline product. This product is a mixture of two isomers. Trituration of this crystalline product in hexane recovered 141.7 mg (10%) of a white crystalline product. This isomer was characterized by NMR and mass spectra to be the (1aα,2β,8bα) isomer **8a**. The hexane filtrate was concentrated in vacuo to give 206 mg of white film which is a mixture of 30% **8a** and 70% **8b** by ¹H NMR.

### Example 6

### cis-3-Butyl-3-ethyl-5-phenyl-2,3,4,5-tetrahydrobenzothiepine-1,1-dioxide (9a), trans-3-Butyl-3-ethyl-5-phenyl-2,3,4,5-tetrahydrobenzothiepine-1,1-dioxide (9b), and 3-Butyl-3-ethyl-4-hydroxy-5-cyclohexylidine-2,3,4,5-tetrahydrobenzothiepine-1,1-dioxide (10)

A mixture of 0.15 g (0.4 mmole) of a 3:7 mixture of **8a** and **8b** was dissolved in 15 ml MeOH in a 3 oz. Fisher/Porter vessel, then was added 0.1 g of 10% Pd/C catalyst. This mixture was hydrogenated at 70 psi H₂ for 5 h and filtered. The filtrate was evaporated to dryness in vacuo to recover 0.117 g of a colorless oil. This material was purified by HPLC eluting with EtOAc-hexane. The first fraction was 4.2 mg (3%) of **9b**. The second fraction, 5.0 mg (4%), was a 50/50 mixture of **9a** and **9b**. The third fraction was 8.8 mg (6%) of **6a**. The fourth fraction was 25.5 mg (18%) of **6b**. The fifth fraction was 9.6 mg (7%) of a mixture of **6b** and a product believed to be 3-butyl-3-ethyl-4,5-dihydroxy-5-phenyl-2,3,4,5-tetrahydrobenzothiepine-1,1-dioxide based on mass spectrum. The sixth fraction was 7.5 mg (5%) of a mixture of **6d** and one of the isomers of **10**, **10a**.

### Example 7

In another experiment, a product (3.7 g) from epoxidation of **3** with excess MCPBA in refluxing CHCl₃ under air was hydrogenated in 100 mL of methanol using 1 g of 10% Pd/C catalyst and 70 psi hydrogen. The product was purified by HPLC to give 0.9 g (25%) of **9b**, 0.45 g (13%) of **9a**, 0.27 g (7%) of **6a**, 0.51 g (14%) of **6b**, 0.02 g (1%) of **6c**, 0.06 g (2%) of one isomer of **10**, **10a** and 0.03 g (1%) of another isomer of **10**, **10b**.

### Example 8

### 2-((2-Benzoylphenylthio)methyl)butyraldehyde (11)

To an ice bath cooled solution of 9.76 g (0.116 mole ) of 2-ethylacrolein in 40 mL of dry THF was added 24.6 g (0.116 mole) of 2-mercaptobenzophenone in 40 mL of THF followed by 13 g (0.128 mole) of triethylamine. The reaction mixture was stirred at room temperature for 3 days , diluted with ether, and was washed successively with dilute HCl, brine, and 1 M potassium carbonate. The ether layer was dried over MgSO₄ and concentrated in vacuo. The residue was purified by HPLC (10% EtOAc-hexane) to give 22 g (64%) of **11** in the second fraction. An attempt to further purifiy this material by kugelrohr distillation at 0.5 torr (160-190 °C) gave a fraction (12.2 g) which contained starting material indicating a reversed reaction during distillation. This material was dissolved in ether (100 mL) and was washed with 50 mL of 1 M potassium carbonate three times to give 6.0 g of a syrup which was purified by HPLC (10% EtOAc-hexane) to give 5.6 g of pure **11**.

### Example 9

### 3-Ethyl-5-phenyl-2,3-dihydrobenzothiepine (12)

To a mixture of 2.61 g (0.04 mole) of zinc dust and 60 mL of DME was added 7.5 g (0.048 mole) of TiCl₃. The reaction mixture was held at reflux for 2 h. A solution of 2.98 g (0.01 mole) of **11** was added dropwise in 1 h. The reaction mixture was held at reflux for 18 h, cooled and poured into water. The organic was extracted into ether. The ether layer was washed with brine and filtered through Celite. The filtrate was dried over MgSO₄ and concentrated. The residual oil (2.5 g) was purified by HPLC to give 2.06 g (77%) of **12** as an oil in the second fraction.

### Example 10

### (1aα,2α,8bα) 2-Ethyl-8b-phenyl-1a,2,3,8b-tetrahydro-benzothiepino-[4,5-b]oxirene-4,4-dioxide (13)

To a solution of 1.5 g (5.64 mmole) of **12** in 25 ml of CHCl₃ was added 6.8 g (19.4 mmole) of 50-60% MCPB portionwise causing an exothem and formation of a white solid. The mixture was stirred at room temperature overnight diluted with 100 ml methylene chloride and washed successively with 10% K₂CO₃ (4x50 ml), water (2x25 ml) and brine. The organic layer was then dried over MgSO₄ and evaporated to dryness to recover 1.47 g of an off white solid. ¹H NMR indicated that only one isomer is present. This solid was slurried in 200 ml of warm Et₂O and filtered to give 0.82 g (46%) of **13** as a white solid, mp 185-186.5 °C.

### Example 11

### (3α,4β,5α)-3-Ethyl-4-hydroxy-5-phenyl-2,3,4,5-tetrahydrobenzothiepine-1,1-dionde (14a), (3α,4β,5β) 3-Ethyl-4-hydroxy 5-phenyl-2,3,4,5-tetrahydrobenzothiepine-1,1-dioxide (14b), and cis-3-Ethyl-5-phenyl-2,3,4,5-tetrahydro-benzothiepine-1,1-diode (15)

A mixture of 0.5 g (1.6 mole) of **13**, 50 ml of acetic acid and 0.5 g of 10% Pd/C catalyst was hydrogenated with 70 psi hydrogen for 4 h. The crude reaction slurry was filtered and the filtrate was stirred with 150 ml of a saturated NaHCO₃ solution followed by 89 g of NaHCO₃ powder portionwise to neutralize the rest of acetic acid. The mixture was extracted with methylene chloride (4x25 ml), then the organic layer was dried over MgSO₄ and concentrated in vacuo to give 0.44 g (87%) of a voluminous white solid which was purified by HPLC (EtOAc-Hexane) to give 26.8 mg (6%) of **15** in the first fraction, 272 mg (54%) of **14a** as a solid, mp 142-143.5 °C, in the second fraction, and 35 mg (7%) of impure **14b** in the third fraction.

### Example 12

### 2-Ethyl-2-((2-Hydroxymethylphenyl)thiomethyl)hexenal (16)

A mixture of 5.0 g (0.036 mole) of 2-mercaptobenzyl alcohol, 6.4 g (0.032 mole) of **1**, 3.6 g (0.036 mole) of triethylamine and 25 mL of 2-methoxyethyl ether was held at reflux for 7 h. Additional 1.1 g of mercaptobenzyl alcohol and 0.72 g of triethylamine was added to the reaction mixture and the mixture was held at reflux for additional 16 h. The reaction mixture was cooled and poured into 6N HCl and extracted with methylene chloride. The methylene chloride extract was washed twice with 10% NaOH, dried over MgSO₄ and concentrated in vacuo to give 9.6 g of residue. Purification by HPLC (20% EtOAc-hexane) gave 3.7 g (41%)of **16** as an oil.

### Example 13

### 2-Ethyl-2-((2-formylphenyl)thiomethyl)hexenal (17)

A mixture of 3.7 g of **16**, 5.6 g (0.026 mole) of pyridinium chlorochromate, 2 g of Celite and 30 mL of methylene chloride was stirred for 18 h and filtered through a bed of silica gel. The silica gel was eluted with methylene chloride. The combined methylene chloride eluant was purified by HPLC (20% ETOAc-hexane) to give 2.4 g (66%) of an oil.

### Example 14

### 3-Butyl-3-ethyl-2,3-dihydrobenzothiepine (18)

A mixture of 2.6 g (0.04 mole) of zinc dust, 7.2 g (0.047 mole) of TiCl₃, and 50 mL of DME was held at reflux for 2 h and cooled to room temperature. To this mixture was added 2.4 g (8.6 mmole) of **17** in 20 mL of DME in 10 min. The reaction mixture was stirred at room temperature for 2 h and held at reflux for 1 h then was let standing at room temperature over weekend. The reaction mixture was poured into dilute HCl and was stirred with methylene chloride. The methylene chloride-water mixture was filtered through Celite. The methylene chloride layer was washed with brine, dried over MgSO₄, and concentrated in vacuo to give 3.0 g of a residue. Purification by HPLC gave 0.41 g (20%) of **18** as an oil in the early fraction.

### Example 15

### (1aα,2α,8bα) 2-Butyl-2-ethyl-1a,2,3,8b-tetrahydrobenzothiepino[4,5-b]oxirene-4,4-dioxide (19a) and (1aα,2β,8bα) 2-Butyl-2-ethyl-8b-phenyl-1a,2,3,8b-tetrahydro-benzothiepino[4,5-b]oxirene-4,4-dioxide (19b)

To a solution of 0.4 g of 0.4 g (1.6 mmole) of **18** in 30 mL of methylene chloride was added 2.2 g (3.2 mmole) of 50-60% MCPBA. The reaction mixture was stirred for 2 h and concentrated in vacuo. The residue was dissolved in 30 mL of CHCl₃ and was held at reflux for 18 h under N₂. The reaction mixture was stirred with 100 mL of 10% NaOH and 5 g of sodium sulfite. The methylene chloride layer was washed with brine, dried over MgSO₄ and concentrated in vacuo. The residue was purified by HPLC (20% EtOAc-hexane) to give a third fraction which was further purified by HPLC (10% EtOAc-hexane) to give 0.12 g of syrup in the first fraction. Recrystallization from hexane gave 0.08 g (17%) of **19a**, mp 89.5-105.5 °C. The mother liquor from the first fraction was combined with the second fraction and was further purified by HPLC to give additional **19a** in the first fraction and 60 mg of **19b** in the second fraction. Crystallization from hexane gave 56 mg of a white solid.

### Example 16

### 3-Butyl-3-ethyl-4,5-dihydroxy-5-phenyl-2,3,4,5-tetrahydrobenzothiepine-1,1-dioxide (20)

This product was isolated along with **6b** from hydrogenation of a mixture of **8a** and **8b**.

### Example 17

### 3-Butyl-3-ethyl-4-hydroxy-5-phenylthio-2,3,4,5-tetrahydrobenzothiepine-1,1-dioxide (21)

A mixture of 25 mg (0.085 mmole) of **19b**, 0.27 g (2.7 mmole) of thiophenol, 0.37 g (2.7 mmole) of potassium carbonate, and 4 mL of DMF was stirred at room temperature under N₂ for 19 h. The reaction mixture was poured into water and extracted with methylene chloride. The methylene chloride layer was washed successively with 10% NaOH and brine, dried over MgSO₄, and concentrated in vacuo to give 0.19 g of semisolid which contain substantial amounts of diphenyl disulfide. This material was purified by HPLC (5% EtOAc-hexane) to remove diphenyl disulfide in the first fraction. The column was then eluted with 20% EtOAc-hexane to give 17 mg of a first fraction, 4 mg of a second fraction and 11 mg of a third fraction which were three different isomers of **21**, i.e. **21a**, **21b**, and **21c**, respectively, by ¹H NMR and mass spectra.

### Example 18

### Alternative Synthesis of 6c and 6d

### A. Preparation from 2-((2-Benzoylphenylthio)methyl)-2-ethylhexanal (2)

### Step 1. 2-((2-Benzoylphenylsulfonyl)methyl)-2-ethylhexanal (44)

To a solution of 9.0 g (0.025 mole) of compound **2** in 100 ml of methylene chloride was added 14.6 g (0.025 mol) of 50-60% MCPBA portionwise. The reaction mixture was stirred at room temperature for 64 h then was stirred with 200 ml of 1 M potassium carbonate and filtered through Celite. The methylene chloride layer was washed twice with 300 ml of 1 M potassium carbonate, once with 10% sodium hydroxide and once with brine. The insoluble solid formed during washing was removed by filtration through Celite. The methylene chloride solution was dried and concentrated in vacuo to give 9.2 g (95%)of semisolid. A portion (2.6 g) of this solid was purified by HPLC(10% ethyl acetate-hexane) to give 1.9 g of crystals, mp 135-136 °C

### Step 2. 2-((2-Benzylphenylsulfonyl)methyl)-2-ethylhexanal (45)

A solution of 50 g (0.13 mole) of crude **44** in 250 ml of methylene chloride was divided in two portions and charged to two Fisher-Porter bottles. To each bottle was charged 125 ml of methanol and 5 g of 10% Pd/C. The bottles were pressurized with 70 psi of hydrogen and the reaction mixture was stirred at room temperature for 7 h before being charged with an additional 5 g of 10% Pd/C. The reaction mixture was again hydrogenated with 70 psi of hydrogen for 7 h. This procedure was repeated one more time but only 1 g of Pd/C was charged to the reaction mixture. The combined reaction mixture was filtered and concentrated in vacuo to give 46.8 g of **45** as brown oil.

### Step 3. (3α,4α,5α) 3-Butyl-3-ethyl-4-hydroxy-5-phenyl-2,3,4,5-tetrahydrobenzothiepine-1,1-dioxide (6c), and (3α,4β,5β) 3-Butyl-3-ethyl-4-hydroxy-5-phenyl-2,3,4,5-tetrahydrobenzothiepine-1,1-dioxide (6d)

To a solution of 27.3 g (73.4 mmole) of **45** in 300 ml of anhydrous THF cooled to 2 °C with an ice bath was added 9.7 g (73.4 mmole) of 95% potassium t-butoxide. The reaction mixture was stirred for 20 min, quenched with 300 ml of 10% HCl and extracted with methylene chloride. The methylene chloride layer was dried over magnesium sulfate and concentrated in vacuo to give 24.7 g of yellow oil. Purification by HPLC (ethyl acetate-hexane) yielded 9.4 g of recovered **45** in the first fraction, 5.5 g (20%) of **6c** in the second fraction and 6.5 g (24%) of **6d** in the third fraction.

### B. Preparation from 2-hydroxydiphenylmethane

### Step 1. 2-mercaptodiphenylmethane (46)

To a 500 ml flask was charged 16 g (0.33 mol) of 60% sodium hydride oil dispersion. The sodium hydride was washed twice with 50 ml of hexane. To the reaction flask was charged 100 ml of DMF. To this mixture was added a solution of 55.2 g (0.3 mol) of 2-hydroxydiphenylmethane in 200 ml of DMF in 1 h while temperature was maintained below 30 °C by an ice-water bath. After complete addition of the reagent, the mixture was stirred at room temperature for 30 min then cooled with an ice bath. To the reaction mixture was added 49.4 g (0.4 mole) of dimethyl thiocarbamoyl chloride at once. The ice bath was removed and the reaction mixture was stirred at room temperature for 18 h before being poured into 300 ml of water. The organic was extracted into 500 ml of toluene. The toluene layer was washed successively with 10% sodium hydroxide and brine and was concentrated in vacuo to give 78.6 g of a yellow oil which was 95% pure dimethyl *O*-2-benzylphenyl thiocarbamate. This oil was heated at 280-300 °C in a kugelrohhr pot under house vacuum for 30 min. The residue was kugelrohr distilled at 1 torr (180-280 °C). The distillate (56.3 g) was crystallized from methanol to give 37.3 g (46%) of the rearranged product dimethyl S-2-benzylphenyl thiocarbamate as a yellow solid. A mixture of 57 g (0.21 mole) of this yellow solid, 30 g of potassium hydroxide and 150 ml of methanol was stirred overnight then was concentrated in vacuo. The residue was diluted with 200 ml of water and extracted with ether. The aqueous layer was made acidic with concentrate HCl, The oily suspension was extracted into ether. The ether extract was dried over magnesium sulfate and concentrated in vacuo. The residue was crystallized from hexane to give 37.1 g (88%) of 2-mercaptodiphenylmethane as a yellow solid.

### Step 2. 2-((2-Benzylphenylthio)methyl)-2-ethylhexanal (47)

A mixture of 60 g (03 mole) of yellow solid from step 1, 70 g (0.3 mole) of compound 1 from preparation 1, 32.4 g (0.32 mole) of triethylamine, 120 ml of 2-methoxyethyl ether was held at reflux for 6 hr and concentrated in vacuo. The residue was triturated with 500 ml of water and 30 ml of concentrate HCl. The organic was extracted into 400 ml of ether. The ether layer was washed successively with brine, 10% sodium hydroxide and brine and was dried over magnesium sulfate and concentrated in vacuo. The residue (98.3 g) was purified by HPLC with 2-5% ethyl acetate-hexane as eluent to give 2-((2-benzylphenylthio)methyl)-2-ethylhexanal **47** as a yellow syrup.

### Step 3. 2-((2-Benzylphenylsulfonyl)methyl)-2-ethylhexanal (45)

To a solution of 72.8 g (0.21 mole) of yellow syrup from step 2 in 1 liter of methylene chloride cooled to 10 °C was added 132 g of 50-60% MCPBA in 40 min. The reaction mixture was stirred for 2 h. An additional 13 g of 50-60% MCPBA was added to the reaction mixture. The reaction mixture was stirred for 2 h and filtered through Celite. The methylene chloride solution was washed twice with 1 liter of 1 M potassium carbonate then with 1 liter of brine. The methylene chloride layer was dried over magnesium sulfate and concentrated to 76 g of 2-((2-benzylphenylsulfonyl)methyl)-2-ethylhexanal **45** as a syrup.

### Step 4. (3α,4α,5α) 3-Butyl-3-ethyl-4-hydroxy-5-phenyl-2,3,4,5-tetrahydrobenzothiepine-1,1-dioxide (6c), and (3α,4β,5β) 3-Butyl-3-ethyl-4-hydroxy-5-phenyl-2,3,4,5-tetrahydrobenzothiepine-1,1-dioxide (6d)

Reaction of **45** with potassium t-butoxide according to the procedure in step 3 of procedure A gave pure **6c** and **6d** after HPLC.

### Example 19

### (3α,4β,5β) 3-Butyl-3-ethyl-4-hydroxy-8-methoxy-5-phenyl-2,3,4,5-tetrahydrobenzothiepine-1,1-dioxide (25) and (3α,4α,5α) 3-Butyl-3-ethyl-4-hydroxy-8-methoxy-5-phenyl-2,3,4,5-tetrahydrobenzothiepine-1,1-dioxide (26)

### Step 1. Preparation of 2-((2-benzoyl-4-methoxy phenylthio)methyl)-2-ethylhexanal (22)

2-Hydroxy-4-methoxybenzophenone was converted to the dimethyl *O*-2-benzoyphenyl thiocarbamate by methods previously described in example 18. The product can be isolated by recrystallization from ethanol. Using this improved isolation procedure no chromatography was needed. The thermal rearrangement was performed by reacting the thiocarbamate( 5 g) in diphenyl ether at 260 °C as previously described. The improved isolation procedure which avoided a chromatography step was described below.

The crude pyrolysis product was then heated at 65 °C in 100 ml of methanol and 100 ml of THF in the presence of 3.5 g of KOH for 4 h. After removing THF and methanol by rotary evaporation the solution was extracted with 5 % NaOH and ether. The base layer was acidified and extracted with ether to obtain a 2.9 g of crude thiophenol product. The product was further purified by titrating the desired mercaptan into base with limited KOH. After acidification and extraction with ether pure 2-mercapto-4-methoxybenzophenone (2.3 g) was isolated.

2-mercapto-4-methoxybenzophenone can readily be converted to the 2-((2-benzoyl-4-methoxyphenylthio)methyl)-2-ethylhexanal (**22**) by reaction with 2-ethyl-2-(mesyloxymethyl)hexanal (**1**) as previously described.

### Step 2. 2-((2-Benzoyl-5-methoxyphenylsulfonyl)methyl)-2-ethylhexanal (23)

Substrate **22** was readily oxidized to **2-((2-benzoyl-5-methoxyphenylsulfonyl)methyl)-2-ethylhexanal (23)** as described in example 18.

### Step 3. 2-((2-benzyl-5-methoxyphenylsulfonyl)methyl)-2-ethylhexanal (24)

Sulfone **23** was then reduced to **2-((2-benzyl-5-methoxyphenylsulfonyl)methyl)-2-ethylhexanal (24)** as described in example 18.

### Step 4. (3α,4β,5β) 3-Butyl-3-ethyl-4-hydrozy-8-methoxy-5-phenyl-2,3,4,5-tetrahydrobenzothiepine-1,1-dioxide (25) and (3α,4α,5α) 3-Butyl-3-ethyl-4-hydroxy-8-methoxy-5-phenyl-2,3,4,5-tetrahydrobenzothiepine-1,1-dioxide (26)

A 3-neck flask equipped with a powder addition funnel,thermocouple and nitrogen bubbler was charged with 19.8 g (0.05 mole) of sulfone **24** in 100 ml dry THF. The reaction was cooled to -1.6 °C internal temperature by means of ice/salt bath. Slowly add 5.61 g (0.05 mole) of potassium t-butoxide by means of the powder addition funnel. The resulting light yellow solution was maintained at -1.6 °C. After 30 min reaction 400 ml of cold ether was added and this solution was extracted with cold 10 % HCl. The acid layer was extracted with 300 ml of methylene chloride. The organic layers were combined and dried over magnesium sulfate and after filtration stripped to dryness to obtain 19.9 g of product. ¹H nmr and glpc indicated a 96% conversion to a 50/50 mixture of **25** and **26**. The only other observable compound was 4% starting sulfone **24**.

The product was then dissolved in 250 ml of 90/10 hexane/ethyl acetate by warming to 50 °C. The solution was allowed to cool to room temperature and in this way pure **26** can be isolated. The crystallization can be enhanced by addition of a seed crystal of **26**. After 2 crystallizations the mother liquor which was now 85.4% **25** and has a dry weight of 8.7 g. This material was dissolved in 100 ml of 90/10 hexane/ethyl acetate and 10 ml of pure ethyl acetate at 40 C. Pure **25** can be isolated by seeding this solution with a seed crystal of **25** after storing it overnight at 0 C.

### Example 20

### (3α,4α,5α) 3-Butyl-3-ethyl-4,8-dihydroxy-5-phenyl-2,3,4,5-tetrahydrobenzothiepine-1,1-dioxide (27)

In a 25 ml round bottomed flask , 1 g of **26**( 2.5 mmoles) and 10 ml methylene chloride were cooled to - 78 °C with stirring. Next 0.7 ml of boron tribromide(7.5 mmole) was added via syringe. The reaction was allowed to slowly warm to room temperature and stirred for 6 h. The reaction was then diluted with 50 ml methylene chloride and washed with saturated NaCl and then water.The organic layer was dried over magnesium sulfate. The product (0.88g) **27** was characterized by NMR and mass spectra.

### Example 21

### General Alkylation of phenol 27

A 25 ml flask was charged with 0.15 g of **27**(0.38 mmole), 5 ml anhydrous DMF, 54 mg of potassium carbonate(0.38 mmole) and 140 mg ethyl iodide (0.9 mmole). The reaction was stirred at room temperature overnight.The reaction was diluted with 50 ml ethyl ether and washed with water (25 ml) then 5% NaOH (20 ml) and then sat. NaCl. After stripping off the solvent the ethoxylated product **28** was obtained in high yield. The product was characterized by NMR and mass spectra.
This same procedure was used to prepare products listed in table 1 from the corresponding iodides or bromides. For higher boiling alkyl iodides and bromides only one equivalent of the alkyl halide was used.

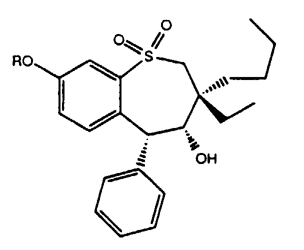

**Table 1**

| **Compound No.** | **R** |
|---|---|
| **27** | H |
| **26** | Me |
| **28** | Et |
| **29** | hexyl |
| **30** | Ac |
| **31** | (CH2)6-N-pthalimide |

### Example 22

### (3α,4α,5α) 3-Butyl-3-ethyl-4-hydroxy-7-hydroxyamino-5-phenyl-2,3,4,5-tetrahydrobenzothiepine-1,1 dioxide (37) and (3α,4β,5β) 3-Butyl-3-ethyl-4-hydroxy-7-hydroxyamino-5-phenyl-2,3,4,5-tetrahydrobenzothiepine-1,1-dioxide (38)

### Step 1. Preparation of 2-chloro-5-nitrodiphenylmethane (32)

### Procedure adapted from reference :Synthesis -Stuttgart 9 770-772 (1986) Olah G. Et al

Under nitrogen, a 3 neck flask was charged with 45 g (0.172 mole ) of 2-chloro-5-nitrobenzophenone in 345 ml methylene chloride and the solution was cooled to ice/water temperature. By means of an additional funnel, 150 g( 0.172 mole) of trifluoromethane sulfonic acid in 345 ml methylene chloride was added slowly. Next 30 g of triethylsilane (0.172 mole) in 345 ml methylene chloride was added dropwise to the chilled solution. Both addition steps( trifluoromethane sulfonic acid and triethylsilane)were repeated. After the additions were completed the reaction was allowed to slowly warm up to room temperature and stirred for 12 h under nitrogen. The reaction mixture was then poured into a chilled stirred solution of 1600 ml of saturated sodium bicarbonate. Gas evolution occurred. Poured into a 4 liter separatory funnel and separated layers. The methylene chloride layer was isolated and combined with two 500 ml methylene chloride extractions of the aqueous layer. The methylene chloride solution was dried over magnesium sulfate and concentrated in vacuo. The residue was recrystallized from hexane to give 39 g product . Structure **32** was confirmed by mass spectra and proton and carbon NMR.

### Step 2. Preparation of 2-((2-benzyl-4-nitrophenylthio)methyl)-2-ethylhexanal (33)

The 2-chloro-5-nitrodiphenylmethane product **32** (40 g, 0.156 mole) from above was placed in a 2 liter 2 neck flask with water condenser. Next 150 ml DMSO and 7.18 g (0.156 mole) of lithium sulfide was added and the solution was stirred at 75 °C for 12 h. The reaction was cooled to room temperature and then 51.7 g of mesylate IV was added in 90 ml DMSO. The reaction mixture was heated to 80 °C under nitrogen. After 12 h monitored by TLC and added more mysylate if necessary. Continued the reaction until the reaction was completed. Next the reaction mixture was slowly poured into a 1900 ml of 5% acetic aqueous solution with stirring, extracted with 4 X 700 ml of ether, and dried over MgSO4. After removal of ether, 82.7 g of product was isolated. The material can be further purified by silica gel chromatography using 95% hexane and 5 % ethyl acetate. If pure mysylate was used in this step there was no need for further purification. The product **33** was characterized by mass spectra and NMR.

### Step 3. Oxidation of the nitro product 33 to the sulfone 2-((2-benzyl-4-nitrophenylsulfonyl)methyl)-2-ethylhexanal (34)

The procedure used to oxidize the sulfide **33** to the sulfone **34** has been previously described.

### Step 4. Reduction of 34 to 2-((2-benzyl-4-hydroxyaminophenylsulfonyl)methyl)-2-ethylhexanal (35)

A 15 g sample of **34** was dissolved in 230 ml of ethanol and placed in a 500 ml rb flask under nitrogen. Next 1.5 g of 10 wt.% Pd/C was added and hydrogen gas was bubbled through the solution at room temperature until the nitro substrate **34** was consumed. The reaction could be readily monitored by silica gel TLC using 80/20 hexane/EtOAc. Product **35** was isolated by filtering off the Pd/C and then stripping off the EtOH solvent. The product was characterized by NMR and mass spectra.

### Step 5. Preparation of the 2-((2-benzyl-4-N,O-di-(t-butoxycarbonyl)hydroxyaminophenylsulfonyl)methyl)-2-ethylhexanal (36).

A 13.35 g sample of **35** (0.0344 mole) in 40 ml of dry THF was stirred in a 250 ml round bottomed flask. Next added 7.52 g (0.0344 mole) of di-t-butyl dicarbonate in 7 ml THF. Heated at 60 °C overnight. Striped off THF and redissolved in methylene chloride. Extracted with 1 % HCl; and then 5% sodium bicarbonate.
The product was further purified by column chromatography using 90/10 hexane/ethyl acetate and then 70/30 hexane/ethyl acetate. The product **36** was obtained (4.12 g) which appeared to be mainly the di-(t-butoxycarbonyl) derivatives by proton NMR.

### Step 6. (3α,4α,5α) 3-Butyl-3-ethyl-4-hydroxy-7-hydroxyamino-5-phenyl-2,3,4,5-tetrahydrobenzothiepine-1,1-dioxide (37) and (3α,4β,5β) 3-Butyl-3-ethyl-4-hydroxy-7-hydroxyamino-5-phenyl-2,3,4,5-tetrahydrobenzothiepine-1,1-dioxide (38)

A 250ml 3-neck round bottomed flask was charged with 4 g of **36** (6.8 mmoles), and 100 ml of anhydrous THF and cooled to -78 °C under a nitrogen atmosphere. Slowly add 2.29 g potassium tert-butoxide(20.4 mmoles) with stirring and maintaining a -78 °C reaction temperature. After 1 h at -78 °C the addition of base was completed and the temperature was brought to -10 °C by means of a ice/salt bath. After 3 h at -10 °C, only trace **36** remained by TLC. Next add 35 ml of deionized water to the reaction mixture at -10 °C and stirred for 5 min. Striped off most of the THF and added to separatory funnel and extracted with ether until all of the organic was removed from the water phase. The combined ether phases were washed with saturated NaCl and then dried over sodium sulfate. The only products by TLC and NMR were the two BOC protected isomers of **37 and 38**. The isomers were separated by silica gel chromatography using 85% hexane and 15 % ethyl acetate; **BOC-37** (0.71 g) and **BOC-38** (0.78 g).

Next the BOC protecting group was removed by reacting 0.87 g of **BOC-38** (1.78 mmoles) with 8.7 ml of 4 M HCl (34.8 mmoles)in dioxane for 30 min. Next added 4.74 g of sodium acetate (34.8 mmoles) to the reaction mixture and 16.5 ml ether and stirred until clear. After transferring to a separatory funnel extracted with ether and water and then dried the ether layer with sodium sulfate. After removing the ether, 0.665 g of **38** was isolated. Isomer **37** could be obtained in a similar procedure.

### Example 23

### (3α,4α,5α) 3-Butyl-3-ethyl-7-(n-hexylamino)-4-hydroxy-5-phenyl-2,3,4,5-tetrahydrobenzothiepine-1,1-dioxide (40) and (3α,4β,5β) 3-Butyl-3-ethyl-7-(n-hexylamino)-4-hydroxy-5-phenyl-2,3,4,5-tetrahydrobenzothiepine-1,1-dioxide (41)

### Step 1. 2-((2-Benzyl-4-(n-hexylamino)phenylsulfonyl)methyl)-2-ethylhexanal (39)

In a Fischer porter bottle weighed out 0.5 g of **34** (1.2 mmoles) and dissolved in 3.8 ml of ethanol under nitrogen. Next added 0.1 g of Pd/C and 3.8 ml of hexanal. Seal and pressure to 50 psi of hydrogen gas. Stirred for 48 h. After filtering off the catalyst and removing the solvent by rotary evaporation **39** was isolated by column chromatography (0.16 g) using 90/10 hexane ethyl acetate and gradually increasing the mobile phase to 70/30 hexane/ethyl acetate. The product was characterized by NMR and mass spectra.

### Step 2. (3α,4α,5α) 3-Butyl-3-ethyl-7-(n-hexylamino)-4-hydroxy-5-phenyl-2,3,4,5-tetrahydrobenzothiepine-1,1-dioxide (40) and (3α,4β,5β) 3-Butyl-3-ethyl-7-(n-hexylamino)-4-hydroxy-5-phenyl-2,3,4,5-tetrahydrobenzothiepine-1,1-dioxide (41)

A 2-neck, 25 ml round bottomed flask with stir bar was charged with 0.158 g **39** (0.335 mmole) and 5 ml anhydrous THF under nitrogen. Cool to -10 °C by means of a salt/water bath. Slowly add 0.113 g of potassium tert butoxide (0.335 mmole). After 15 min at -10 °C all of the starting material was consumed by TLC and only the two isomers **40** and **41** were observed. Next added 5 ml of chilled 10% HCl and stirred at -10 °C for 5 min. Transferred to a separatory funnel and extract with ether. Dried over sodium sulfate. Proton NMR of the dried product (0.143 g) indicated only the presence of the two isomers **40** and **41**. The two isomers were separated by silica gel chromatography using 90/10 hexane ethyl acetate and gradually increasing the mobile phase to 70/30 hexane/ethyl acetate. **40** ( 53.2 mg); **41**(58.9 mg).

### Example 24

### Quaternization of amine substrates 40 and 41

Amine products such as **40** and **41** can be readily alkylated to quaternary salts by reaction with alkyl halides. For example **40** in DMF with 5 equivalents of methyl iodide in the presence of 2,6 dimethyl lutidine produces the dimethylhexylamino quaternary salt.

### Example 25

### (3α,4β,5β) 3-Butyl-3-ethyl-4-hydroxy-5-(4-iodophenyl)-2,3,4,5-tetrahydrobenzothiepine-1,1-dioxide (42)

In a 25 ml round bottomed flask 0.5 g (1.3 mmole) of **6d** , 0.67 g of mercuric triflate were dissolved in 20 ml of dry methylene chloride with stirring. Next 0.34 g of Iodine was added and the solution was stirred at room temperature for 30 h. The reaction was then diluted with 50 ml methylene chloride and washed with 10 ml of 1 M sodium thiosulfate; 10 ml of saturated KI ; and dried over sodium sulfate. See Tetrahedron, Vol.50, No. 17, pp 5139-5146 (1994) Bachki, F. Et al.Mass spectrum indicated a mixture of **6d** , mono iodide **42** and a diiodide adduct. The mixture was separated by column chromatography and **42** was characterized bt NMR and mass spectra.

### Example 26

### (3α,4β,5β) 3-Butyl-5-(4-carbomethoxyphenyl)-3-ethyl-4-hydroxy-2,3,4,5-tetrahydrebenzothiepine-1,1-dioxide (43)

A 0.1 g sample of **42** ( 0.212 mmole), 2.5 ml dry methanol, 38 µl triethylamine (0.275 mmole), 0.3 ml toluene and 37 mg of palladium chloride (0.21 mmole) was charged to a glass lined mini reactor at 300 psi carbon monoxide. The reaction was heated at 100 °C overnight. The catalyst was filtered and a high yield of product was isolated.
The product was characterized by NMR and mass spectra.

Note the ester functionalized product **43** can be converted to the free acid by hydrolysis.

### Example 27

### (3α,4α,5α) 3-Butyl-3-ethyl-4-hydroxy-7-methoxy-5-phenyl-2,3,4,5-tetrahydrobenzothiepine-1,1-dioxide (48), and (3α,4β,5β) 3-Butyl-3-ethyl-4-hydroxy-7-methoxy-5-phenyl-2,3,4,5-tetrahydrobenzothiepine-1,1-dioxide (49)

### Step 1. 2-Mercapto-5-methoxybenzophenone (50)

Reaction of 66.2 g of 4-methoxythiophenol with 360 ml of 2.5 N n-butyllithium, 105 g of tetramethylethylenediamine and 66.7 g of benzonitrile in 600 ml cyclohexane according to the procedure in WO 93/16055 gave 73.2 g of brown oil which was kugelrohr distilled to remove 4-methoxythiophenol and gave 43.86 g of crude **50** in the pot residue.

### Step 2. 2-((2-Benzoyl-4-methoxyphenylthio)methyl)-2-ethylhexanal (51)

Reaction of 10 g (0.04 mole) of crude **50** with 4.8 g (0.02 mole)of mesylate **1** and 3.2 ml (0.23 mole) of triethylamine in 50 ml of diglyme according to the procedure for the preparation of **2** gave 10.5 g of crude product which was purified by HPLC (5% ethyl acetate-hexane) to give 1.7 g (22%) of **51**.

### Step 3. 2-((2-Benzoyl-4-methoxyphenylsulfonyl)methyl)-2-ethylhexanal (52)

A solution of 1.2 g (3.1 mmoles) of **51** in 25 ml of methylene chloride was reacted with 2.0 g (6.2 mmoles) of 50-60% MCPBA according to the procedure of step 2 of procedure A in example 18 gave 1.16 g (90%) of **52** as a yellow oil.

### Step 4. 2-((2-Benzyl-4-methoxyphenylsulfonyl)methyl)-2-ethylhexanal (53)

Hydrogenation of 1.1 g of **52** according to the procedure of step 3 of procedure A of example 18 gave **53** as a yellow oil (1.1 g).

### Step 5. (3α,4α,5α) 3-Butyl-3-ethyl-4-hydroxy-7-methoxy-5-phenyl-2,3,4,5-tetrahydrobenzothiepine-1,1-dioxide (48), and (3α,4β,5β) 3-Butyl-3-ethyl-4-hydroxy-7-methoxy-5-phenyl-2,3,4,5-tetrahydrobenzothiepine-1,1-dioxide (49)

A solution of 1.1 g of **53**, 0.36 g of potassium t-butoxide and 25 ml of anhydrous THF was held at reflux for 2 h and worked up as in step 4 of procedure A of example 18 to give 1.07 g of a crude product which was purified by HPLC to give 40 mg (4%) of **48** as crystals, mp 153-154 °C and 90 mg (8%) of **49** as solid, mp 136-140 °C.

### Example 28

### 5-Phenyl-2,3-dihydrospirobenzothiepine-3,1'-cyclohexane (57)

### Step 1.1-(Hydroxymethyl)-cyclohexanecarboxaldehyde (54)

To a cold (O °C) mixture of 100 g (0.891 mole) of cyclohexanecarboxaldehyde, 76.5 g of 37% of formaldehyde in 225 ml of methanol was added dropwise 90 ml of 1 N Sodium hydroxide in 1 h. The reaction mixture was stirred at room temperature over **48** then was evaporated to remove methanol. The reaction mixture was diluted with water and extracted with methylene chloride. The organic layer was washed with water, brine, and dried over sodium sulfate and concentrated under vacuum to give 75 g (59.7%) of thick oil. Proton NMR and mass spectra were consistent with the product.

### Step 2.1-(mesyloxymethyl)cyclohexanecarboxaldehyde (55)

To a cold (0 °C) mixture of alcohol **54** (75 g, 0.54 mole) and 65.29 g (0.57 mole) of methanesulfonyl chloride in 80 ml of methylene chloride was added a solution of pyridine (47.96 g, 0.57 mole) in 40 ml of methylene chloride. The reaction mixture was stirred at room temperature for 18 h then quenched with water, acidified with conc. HCl and extracted with methylene chloride. The organic layer was washed with water, brine, and dried over sodium sulfate and concentrated under vacuum to give 91.63 g (77.8%) of thick oil. Proton NMR and mass spectra were consistent with the product.

### Step 3. 1-((2-Benzoylphenylthio)methyl)cyclohexanecarboxaldehyde (56)

A mixture of 69 g (0.303 mole) of 2-mercaptobenzophenone, 82 g (0.303 mole) of mesylate **55**, 32 g of triethylamine, and 150 ml of diglyme was stirred and held at reflux for 24 h. The mixture was cooled, poured into dil. HCl and extracted with methylene chloride. The organic layer was washed with 10% NaOH, water, brine, and dried over sodium sulfate and concentrated under vacuum to remove excess diglyme. This was purified by silica gel flush column (5% EtOAc: Hexane) and gave 18.6 g (75.9%) of yellow oil. Proton NMR and mass spectra were consistent with the product.

### Step 4. 5-Phenyl-2,3-dihydrospirobenzothiepine-3,1'-cyclohexane (57)

To a mixture of 6.19 g of zinc dust and 100 ml of dry DME was added TiCl₃(16.8 g, 0.108 mole) . The reaction mixture was heated to reflux for 2 h. A solution of compound **56** (8.3 g, 0.023 mole) in 50 ml of DME was added dropwise to the reaction mixture in 1 h and the mixture was held at reflux for 18 h. The mixture was cooled, poured into water and extracted with ether. The organic layer was washed with water, brine, and dried over sodium sulfate, filtered through celite and concentrated under vacuum. The residue was purified by HPLC (10% EtOAc: Hexane) to give 4.6 g (64%) of white solid, mp 90-91 °C. Proton and carbon NMR and mass spectra were consistent with the product.

### Example 29

### 8b-Phenyl-1a,2,3,8b-tetrahydrospiro(benzothiepino[4,5-b]oxirene-2,1'-cyclohexane)-4,4-dioxide (58)

To a solution of **57** (4.6 g, 15 mmole) in 50 ml chloroform under nitrogen was added 55% MCPBA (16.5 g, 52.6 mmole) portionwise with spatula. The reaction was held at reflux for 18 h and washed with 10% NaOH(3X), water, brine, and dried over sodium sulfate and concentrated under vacuum to give 5 g of crude product. This was recrystallized from Hexane/EtOAc to give 4.31 g (81%) of yellow solid, mp 154-155 °C. Proton and carbon NMR and mass spectra were consistent with the product.

### Example 30

### trans-4-Hydroxy-5-phenyl-2,3,4,5-tetrahydro spiro(benzothiepine-3,1'-cyclohexane)-1,1-dioxide (59)

A mixture of 0.5 g (1.4 mmoles) of **58** , 20 ml of ethanol,10 ml of methylene chloride and 0.4 g of 10% Pd/C catalyst was hydrogenated with 70 psi hydrogen for 3 h at room temperature. The crude reaction slurry was filtered through Celite and evaporated to dryness. The residue was purified by HPLC (10% EtOAc-Hexane, 25% EtOAc-Hexane). The first fraction was 300 mg (60%) as a white solid, mp 99-100 °C. Proton NMR showed this was a trans isomer. The second fraction gave 200 mg of solid which was impure cis isomer.

### Example 31

### cis-4-Hydroxy-5-phenyl-2,3,4,5-tetrahydro spiro(benzothiepine-3,1'-cyclohexane)-1,1-dioxide (60)

To a solution of 0.2 g (0.56 mmole) of **59** in 20 ml of CH₂Cl₂, was added 8 g of 50% NaOH and one drop of Aliquat-336 (methyltricaprylylammonium chloride) phase transfer catalyst. The reaction mixture was stirred for 10 h at room temperature. Twenty g of ice was added to the mixture and the mixture was extracted with CH₂Cl₂ (3x10 ml) washed with water, brine and dried over MgSO₄ and concentrated in vacuo to recover 0.15 g of crude product. This was recrystallized from Hexane/EtOAc to give 125 mg of white crystal, mp 209-210 °C . Proton and carbon NMR and mass spectra were consistent with the product.

### Example 32

### (3α,4α,5α) 3-Butyl-3-ethyl-4-hydroxy-5-phenyl-2,3,4,5-tetrahydrobenzothiepine (61), and (3α,4β,5β) 3-Butyl-3-ethyl-4-hydroxy-5-phenyl-2,3,4,5-tetrahydrobenzothiepine (62)

To a solution of 0.5 g (1.47 mmole) of compound **47** in 5 ml of anhydrous THF was added 0.17 g (1.47 mmole) of 95% potassium t-butoxide. The reaction mixture was stirred at room temperature for 18 h and quenched with 10 ml of 10% HCl. The organic was extracted into methylene chloride. The methylene chloride extract was dried over magnesium sulfate and concentrated in vacuo. The residue was purified by HPLC (2% EtOAc-hexane) to give 47 mg of **61** in the second fraction and 38 mg of **62** in the third fraction. Proton NMR and mass spectra were consistent with the assigned structures.

### Example 33

### (3α,4α,5α) 3-Butyl-3ethyl-4-hydroxy-7-amino-5-phenyl-2,3,4,5-tetrahydrobenzothiepine-1,1-dioxide (63) and (3α,4β,5β) 3-Butyl-3-ethyl-4-hydroxy-7-amino-5-phenyl-2,3,4,5-tetrahydrobenzothiepine-1,1-dioxide (64)

An autoclave was charged with 200 mg of **37** in 40 cc ethanol and .02 g 10 % Pd/C. After purging with nitrogen the clave was charged with 100 psi hydrogen and heated to 55 C. The reaction was monitored by TLC and mass spec and allowed to proceed until all of **37** was consumed. After the reaction was complete the catalyst was filtered and the solvent was removed in vacuo and the only observable product was amine **63**. This same procedure was used to produce **64** from **38**.

The examples herein can be repeated with similar success by substituting the generically or specifically described reactants and/or operating conditions of this invention for those used in the preceding examples.

### DOSAGE FORMS

The bile uptake inhibitor compounds of the formula I of this invention can be administered for the prophylaxis and treatment of hyperlipemic diseases or conditions by any means, preferably oral, that produces contact of the compound I with the compound's site of action in the body, for example, preferably in the ileum of a mammal, preferably human.

These agents can be administered by any conventional means available for use in conjunction with pharmaceuticals, either as individual therapeutic agents or in a combination of therapeutic agents.

The amount of a compound of formula I which is required to achieve the desired biological effect will, of course, depend on a number of factors such as the specific compound chosen, the use for which it is intended, the mode of administration and the clinical condition of the recipient.

In general, a daily dose is in the range of from 0.3 to 100 mg (typically from 3 mg to 50 mg) per day per kilogram bodyweight, for example, 3-10 mg/kg/day. This total daily dose is administered to the mammal, preferably human, in single or divided doses, preferably in divided doses 1 to 6 times a day or in sustained release form effective to obtain desired results.

An intravenous dose can, for example, be in the range of from 0.3 mg to 1.0 mg/kg, which is conveniently administered as an Oinfusion of from 10 ng to 100 ng per kilogram per minute. Infusion fluids suitable for this purpose can contain, for example, from 0.1 ng to 10 mg, typically from 1 ng to 10 mg, per milliliter. Unit doses can contain, for example, from 1 mg to 10 g of the compound of the formula I. This ampoules for injection can contain, for example, from 1 mg to 100 mg and orally administrable unit dose formulations, such as tablets or capsules, may contain, for example from 1.0 to 1000 mg, typically from 10 to 500 mg. In the case of pharmaceutically acceptable salts, the weights indicated above refer to the weight of the benzothiepine ion derived from the salt.

For the prophylaxis or treatment of the conditions referred to above, the compounds of formula I can be used as the compound per se, but are preferably presented with an acceptable carrier in the form of a pharmaceutical composition. The carrier must, of course, be acceptable in the sense of being compatible with the other ingredients of the composition and must not be deleterious to the recipient. The carrier can be a solid or a liquid, or both, and is preferably formulated with the compound as a unit-dose composition, for example, a tablet, which can contain from 0.05% to 95% by weight of the active compound. Other pharmacologically active substances can also be present including other compounds of formula I. The pharmaceutical compositions of the invention can be prepared by any of the well known techniques of pharmacy consisting essentially of admixing the components.

Pharmaceutical compositions according to the present invention include those suitable for oral, rectal, topical, buccal (e.g. sublingual) and parenteral (e.g. subcutaneous, intramuscular, intradermal, or intravenous) administration, although the most suitable route in any given case will depend on the nature and severity of the condition being treated and on the nature of the particular compound of formula I which is being used. Enteric-coated and enteric-coated controlled release formulations are also within the scope of the invention. Suitable enteric coatings include cellulose acetate phthalate, polyvinylacetate phthalate, hydroxypropylmethylcellulose phthalate and anionic polymers of methacrylic acid and methacrylic acid methyl ester.

Pharmaceutical compositions suitable for oral administration can be presented in discrete units, such as capsules, cachets, lozenges, or tablets, each containing a predetermined amount of a compound of formula I; as a powder or granules; as a solution or a suspension in an aqueous or non-aqueous liquid; or as an oil-in-water or water-in-oil emulsion. As indicated, such compositions can be prepared by any suitable method of pharmacy which includes the step of bringing into association the active compound and the carrier (which can constitute one or more accessory ingredients). In general, the compositions are prepared by uniformly and intimately admixing the active compound with a liquid or finely divided solid carrier, or both, and then, if necessary, shaping the product. For example, a tablet can be prepared by compressing or molding a powder or granules of the compound, optionally with one or more assessory ingredients. Compressed tablets can be prepared by compressing, in a suitable machine, the compound in a free-flowing form, such as a powder or granules optionally mixed with a binder, lubricant, inert diluent and/or surface active/dispersing agent(s). Molded tablets can be made by molding, in a suitable machine, the powdered compound moistened with an inert liquid diluent.

Pharmaceutical compositions suitable for buccal (sub-lingual) administration include lozenges comprising a compound of formula I in a flavored base, usually sucrose and acacia or tragacanth, and pastilles comprising the compound in an inert base such as gelatin and glycerin or sucrose and acacia.

Pharmaceutical compositions suitable for parenteral administration conveniently comprise sterile aqueous preparations of a compound of formula I. preferably isotonic with the solid of the intended recipient. These preparations are preferably administered intravenously, although administration can also be effected by means of subcutaneous, intramuscular, or intradermal injection. Such preparations can conveniently be prepared by admixing the compound with water and rendering the resulting solution sterile and isotonic with the blood. Injectable compositions according to the invention will generally contain from 0.1 to 5% w/w of the compound of the formula I.

Pharmaceutical compositions suitable for rectal administration are preferably presented as unit-dose suppositories. These can be prepared by admixing a compound of formula I with one or more conventional solid carriers, for example, cocoa butter, and then shaping the resulting mixture.

Pharmaceutical compositions suitable for topical application to the skin preferably take the form of an ointment, cream, lotion, paste, gel, spray, aerosol, or oil. Carriers which can be used include vaseline, lanoline, polyethylene glycols, alcohols, and combinations of two or more thereof. The active compound is generally present at a concentration of from 0.1 to 15% w/w of the composition, for example, from 0.5 to 2%.

Transdermal administration is also possible. Pharmaceutical compositions suitable for transdermal administration can be presented as discrete patches adapted to remain in intimate contact with the epidermis of the recipient for a prolonged period of time. Such patches suitably contain the compound of the formula I in an optionally buffered, aqueous solution, dissolved and/or dispersed in an adhesive, or dispersed in a polymer. A suitable concentration of the active compound is about 1% to 35%, preferably about 3% to 15%. As one particular possibility, the compound of the formula I can be delivered from the patch by electrotransport or iontophoresis, for example, as described in Pharmaceutical Research, 3(6), 318 (1986).

The amount of active ingredient that may be combined with the carrier materials to produce a single dosage form will vary depending upon the host treated and the particular mode of administration.

The dosage regimen to give relief from or ameliorate a disease condition (ie, prophylaxis or treatment) having hyperlipemia as an element of the disease, such as atherosclerosis or protecting against or treating the further high cholesterol plasma or blood levels with the compounds and/or compositions of this invention is selected in accordance with a variety of factors, including the type, age, weight, sex, diet and medical condition of the patient, the severity of the disease, the route of administration, pharmacological considerations such as the activity, efficacy, pharmacokinetics and toxicology profiles of the particular compound employed, whether a drug delivery system is utilized and whether the compound is administered as part of a drug combination. Thus, the dosage regimen actually employed may vary widely and therefore deviate from the preferred dosage regimen set forth above.

Injectable preparations, for example, sterile injectable aqueous or oleaginous suspensions may be formulated according to the known art using suitable dispersing or setting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The solid dosage forms for oral administration including capsules, tablets, pills, powders, and granules noted above are the compound of the formula I admixed with at least one inert diluent such as sucrose lactose or starch. Such dosage forms may also comprise, as in normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

Pharmaceutically acceptable carriers encompass all the foregoing and the like.

### BIOLOGICAL ASSAYS

The utility of the compounds of the present invention is shown by the following assays. These assays are performed in vitro and in animal models essentially using a procedure recognized to show the utility of the present invention.

### In Vitro Assay of compounds that inhibit IBAT-mediated uptake of [¹⁴C]-Taurocholate (TC) in H14 Cells

Baby hamster kidney cells (BHK) transfected with the cDNA of human IBAT (H14 cells) are seeded at 60,000 cells/well in 96 well Top-Count tissue culture plates for assays run within in 24 hours of seeding, 30,000 cells/well for assays run within 48 hours, and 10,000 cells/well for assays run within 72 hours.

On the day of assay, the cell monolayer is gently washed once with 100 µl assay buffer (Dulbecco's Modified Eagle's medium with 4.5 g/L glucose + 0.2% (w/v) fatty acid free bovine serum albumin-(FAF)BSA). To each well 50 µl of a two-fold concentrate of test compound in assay buffer is added along with 50 µl of 6 µM [¹⁴C]-taurocholate in assay buffer (final concentration of 3 µM [¹⁴C]-taurocholate). The cell culture plates are incubated 2 hours at 37° C prior to gently washing each well twice with 100 µl 4° C Dulbecco's phosphate-buffered saline (PBS) containing 0.2% (w/v) (FAF)BSA. The wells are then gently washed once with 100 µl 4° C PBS without (FAF)BSA. To each 200 µl of liquid scintillation counting fluid is added, the plates are heat sealed and shaken for 30 minutes at room temperature prior to measuring the amount of radioactivity in each well on a Packard Top-Count instrument.

### In Vitro Assay of compounds that inhibit uptake of [¹⁴C]-Alanine

The alanine uptake assay is performed in an identical fashion to the taurocholate assay, with the exception that labeled alanine is substituted for the labeled taurocholate.

### In Vivo Assay of compounds that inhibit Rat Ileal uptake of [¹⁴C]-Taurocholate into Bile (See"Metabolism of 3α,7β-dihydroxy-7α-methyl-5β-cholanoic acid and 3α,7β-dihydroxy-7α-methyl-5β-cholanoic acid in hamsters" in Biochimica et Biophysica Acta 833 (1985) 196-202 by Une et al.)

Male wistar rats (200-300 g) are anesthetized with inactin @100 mg/kg. Bile ducts are cannulated with a 10" length of PE10 tubing. The small intestine is exposed and laid out on a gauze pad. A canulae (1/8" luer lock, tapered female adapter) is inserted at 12 cm from the junction of the small intestine and the cecum. A slit is cut at 4 cm from this same junction (utilizing a 8 cm length of ileum). 20 ml of warm Dulbecco's phosphate buffered saline, pH 6.5 (PBS) is used to flush out the intestine segment. The distal opening is cannulated with a 20 cm length of silicone tubing (0.02" I.D. x 0.037" O.D.) The proximal cannulae is hooked up to a peristaltic pump and the intestine is washed for 20 min with warm PBS at 0.25 ml/min. Temperature of the gut segment is monitored continuously. At the start of the experiment, 2.0 ml of control sample ([¹⁴C]-taurocholate @ 0.05 mi/ml with 5 mM cold taurocholate) is loaded into the gut segment with a 3 ml syringe and bile sample collection is begun. Control sample is infused at a rate of 0.25 ml/min for 21 min. Bile samples fractions are collected every 3 minute for the first 27 minutes of the procedure. After the 21 min of sample infusion, the ileal loop is washed out with 20 ml of warm PBS (using a 30 ml syringe), and then the loop is washed out for 21 min with warm PBS at 0.25 ml/min. A second perfusion is initiated as described above but this with test compound being administered as well (21 min administration followed by 21 min of wash out) and bile sampled every 3 min for the first 27 min. If necessary, a third perfusion is performed as above that typically contains the control sample.

### Measurement of Hepatic Cholesterol Concentration (HEPATIC CHOL)

Liver tissue was weighed and homogenized in chloroform:methanol (2:1). After homogenization and centrifugation the supernatant was separated and dried under nitrogen. The residue was dissolved in isopropanol and the cholesterol content was measured enzymatically, using a combination of cholesterol oxidase and peroxidase, as described by Allain, C. A., *et al.* (1974) *Clin. Chem.* **20**, 470.

### Measurement of Hepatic HMG CoA-Reductase Activity (HMG COA)

Hepatic microsomes were prepared by homogenizing liver samples in a phosphate/sucrose buffer, followed by centrifugal separation. The final pelleted material was resuspended in buffer and an aliquot was assayed for HMG CoA reductase activity by incubating for 60 minutes at 37° C in the presence of ¹⁴C-HMG-CoA (Dupont-NEN). The reaction was stopped by adding 6N HCl followed by centrifugation. An aliquot of the supernatant was separated, by thin-layer chromatography, and the spot corresponding to the enzyme product was scraped off the plate, extracted and radioactivity was determined by scintillation counting. (Reference: Akerlund, J. and Bjorkhem, I. (1990) *J. Lipid Res.* **31**, 2159).

### Determination of Serum Cholesterol (SER.CHOL. HDL-CHOL. TGI and VLDL + LDL)

Total serum cholesterol (SER.CHOL) was measured enzymatically using a commercial kit from Wako Fine Chemicals (Richmond, VA); Cholesterol C11, Catalog No. 276-64909. HDL cholesterol (HDL-CHOL) was assayed using this same kit after precipitation of VLDL and LDL with Sigma Chemical Co. HDL Cholesterol reagent, Catalog No. 352-3 (dextran sulfate method). Total serum triglycerides (blanked) (TGI) were assayed enzymatically with Sigma Chemical Co. GPO-Trinder, Catalog No. 337-B. VLDL and LDL (VLDL + LDL) cholesterol concentrations were calculated as the difference between total and HDL cholesterol.

### Measurement of Hepatic Cholesterol 7-α-Hydroxylase Activity (7a-OHase)

Hepatic microsomes were prepared by homogenizing liver samples in a phosphate/sucrose buffer, followed by centrifugal separation. The final pelleted material was resuspended in buffer and an aliquot was assayed for cholesterol 7-α-hydroxylase activity by incubating for 5 minutes at 37° C in the presence of NADPH. Following extraction into petroleum ether, the organic solvent was evaporated and the residue was dissolved in acetonitrile/methanol. The enzymatic product was separated by injecting an aliquot of the extract onto a C₁₈ reversed phase HPLC column and quantitating the eluted material using UV detection at 240nm. (Reference: Horton, J. D., *et al.* (1994) *J. Clin. Invest.* **93**, 2084).

### Measurement of Fecal Bile Acid Concentration (FBA)

Total fecal output from individually housed hamsters was collected for 24 or 48 hours, dried under a stream of nitrogen, pulverized and weighed. Approximately 0.1 gram was weighed out and extracted into an organic solvent (butanol/water). Following separation and drying, the residue was dissolved in methanol and the amount of bile acid present was measured enzymatically using the 3α-hydroxysteroid steroid dehydrogenase reaction with bile acids to reduce NAD. (Reference: Mashige, F., *et al.* (1981) Clin. Chem. **27**, 1352).

### [³H]taurocholate Uptake in Rabbit Brush Border Membrane Vesicles (BBMV)

Rabbit Ileal brush border membranes were prepared from frozen ileal mucosa by the calcium precipitation method describe by Malathi *et al.* (Reference: (1979) *Biochimica Biophysica Acta*, **554**, 259). The method for measuring taurocholate was essentially as described by Kramer *et al.* (Reference: (1992) *Biochimica Biophysica Acta,* **1111**, 93) except the assay volume was 200 µl instead of 100 µl. Briefly, at room temperature a 190 µl solution containing 2µM [³H]-taurocholate(0.75 µCi), 20 mM tris, 100 mM NaCl, 100 mM mannitol pH 7.4 was incubated for 5 sec with 10 µl of brush border membrane vesicles (60-120 µg protein). The incubation was initiated by the addition of the BBMV while vortexing and the reaction was stopped by the addition of 5 ml of ice cold buffer (20 mM Hepes-tris, 150 mM KCl) followed immediately by filtration through a nylon filter (0.2 µm pore) and an additional 5 ml wash with stop buffer.

### Acyl-CoA:cholesterol Acyl Transferase (ACAT)

Hamster liver and rat intestinal microsomes were prepared from tissue as described previously (Reference: (1980) *J. Biol. Chem.* **255**, 9098) and used as a source of ACAT enzyme. The assay consisted of a 2.0 ml incubation containing 24 µM Oleoyl-CoA (0.05 µCi) in a 50 mM sodium phosphate, 2 mM DTT ph 7.4 buffer containing 0.25 % BSA and 200 µg of microsomal protein. The assay was initiated by the addition of oleoyl-CoA. The reaction went for 5 min at 37° C and was terminated by the addition of 8.0 ml of chloroform/ methanol (2:1). To the extraction was added 125 µg of cholesterol oleate in chloroform methanol to act as a carrier and the organic and aqueous phases of the extraction were separated by centrifugation after thorough vortexing. The chloroform phase was taken to dryness and then spotted on a silica gel 60 TLC plate and developed in hexane/ethyl ether (9:1). The amount of cholesterol ester formed was determined by measuring the amount of radioactivity incorporated into the cholesterol oleate spot on the TLC plate with a Packard instaimager.

Data from each of the noted compounds in the assays described above is as set forth in TABLES 2, 3, 4 and 5 as follows:

**TABLE 2**

| COMPOUND | IC50 uM∗ | In vitro % Inhibition of TC Uptake @ 100 uM # | % Inhibition of Alanine Uptake @ 100 uM # | % of Control Transport of TC in Rat Ileum @ 0.1mM # | |
|---|---|---|---|---|---|
| Benzothiaze pine= | 2 | | 0 | 45.5 +/- 0.7 | |
| 12 | | 25 | | | |
| 3 | | 0 | | | |
| 4a | | 3 | | | |
| 5a | | 34 | | | |
| 5b | 40 | | 0 | 72.9 ± 5.4 @ 0.5 mM | |
| 4b | | 9 | | | |
| 18 | | 6 | | | |
| 14b | | 18 | | | |
| 14a | | 13 | | | |
| 13 | | 23 | | | |
| 15 | 60 | | | | |
| 19a | | 0 | | | |
| 19b | | 15 | | | |
| 8a | | 41 | | | |
| Mixture of 8a and 8b | | 69 | | | |
| Mixture of 9a and 9b | 6 | | | | |
| 6a | 5 | | | | |
| 6b | | 85 | | | |
| 9a | 5 | | 0 % @ 25 µM | 53.7 +/- 3.9 | |
| Mixture of 6a and 20 | 13 | | | | |
| Mixture of 6d and 10a | 0.8 | | 14 % @ 25 µM | | |
| 21a | | 37 | | | |
| 21c | | 52 | | | |
| 21b | | 45 | | | |
| 6c | 2 | | 58.5 | 68.8 +/- 5.7 at 0.4 mM | |
| 6d | 0.6 | | 77.7 | 16.1 +/-1.1 @ 0.5 mM 30.2 +/- 0.9 @ 0.15 mM | |
| 17 | | 10 | | | |
| 7 | 50 | | 49.3 | | |
| 10a | 7 | | 77.6 | 62.4 =/- 2.5 @ 0.2 mM | |
| 10b | 15 | | 68.6 | | |
| 25 | 0.1 | | 4 % @ 10 µM | 26.0 +/- 3.3 | |
| 26 | 2 | | 31 % @ 25 µM | 87.9 +/- 1.5 | |
| 27 | 5 | | 7 % @ 20 µM | | |
| 28 | 8 | | 31 % @ 20µM | | |
| 29 | | 88 @ 50 µM | | | |
| 30 | | 96 @ 50 µM | | | |
| 31 | | 41 @ 50 µM | | | |
| 37 | 3 | | 0 % @ 5µM | | |
| 38 | 0.3 | | 11 % @ 5 µM | 20.6 +/- 5.7 | |
| 40 | | 49 @ 50 µM | | | |
| 41 | 2 | | 0 % @ 20µM | | |
| 42 | 1.5 | | | | |
| 43 | 1.5 | | 16 % @ 25 µM | | |
| 48 | 2 | | 22 % @ 20 µM | | |
| 49 | 0.15 | | 21 % @ 200 µM | 21.2 +/- 2.7 | |
| 57 | | 51 @ 50µM | | | |
| 58 | | 20 @ 50µM | | | |
| 59 | 70 | | | | |
| 60 | 9 | | 59 | | |
| 61 | 30 | | 175 | | |
| 62 | 10 | | | | |
| 63 | | 90 @ 6µM | | | |
| 64 | | 100 @ 6µM | | | |

| | | | | | |
|---|---|---|---|---|---|
| ∗ In vitro Taurocholate Cell Uptake | | | | | |
| # Unless otherwise noted | | | | | |
| = Comparative Example is Example No. 1 in WO 93/16055 | | | | | |

**TABLE 3**

| Compound | TC-uptake | TC-uptake | TC-uptake | ACAT | ACAT |
|---|---|---|---|---|---|
| | (H14 cells) | Ileal Loop | (BBMV) | (liver) | (intestine) |
| | *IC(50)* | *EC(50)* | *IC(50)* | *IC(50)* | *IC(50)* |
| COMP.EXAMPLE* | 1 µM | 74 µM | 3 µM | 20 µM | 20 µM |
| 6d | 0.6 µM | 31 µM | 1.5 µM | 25 µM | 20 µM |
| 38 | 0.3 µM | 12 µM | 2 µM | 15 µM | N.D. |
| 49 | 0.1 µM | 12 µM | N.D. | 6 µM | N.D. |
| 25 | 0.1 µM | 20 µM | 0.8 µM | 8 µM | 8 µM |
| Comparative Example is Example No. 1 in WO 93/16055 | | | | | |

**TABLE 4.**

| **EFFICACY OF COMPOUND NO. 25 IN CHOLESTEROL-FED HAMSTERS** | | | |
|---|---|---|---|
| **PARAMETER** | **CONTROL** | **4% CHOLESTYRAMINE** | **0.2% CPD. NO. 25** |
| WEIGHT (G) | *(mean ± SEM, *p<0.005, A-Student's t, B-Dunnett's)* | | |
| day 1 | 117(2) | 114(6) | 117(5) |
| day 14 | 127(3) | 127(3) | 132(4) |
| LIVER WEIGHT (G) | 5.4(0.3) | 4.9(0.4) | 5.8(0.2) |
| SER.CHOL(mg%) | 143(7) | 119(4)*A,B | 126(2)*A,B |
| HDL-CHOL(mg%) | 89(4) | 76(3)*A,B | 76(1)*A,B |
| VLDL + LDL | 54(7) | 42(3)*A | 50(3) |
| TGI(mg%) | 203(32) | 190(15) | 175(11) |
| HEPATIC CHOL(mg/g) | 25 (0.3) | 1.9 (0.1)*A,B | 1.9 (0.1)*A,B |
| HMG COA pm/mg/min | 15.8(7.6) | 448.8(21.6)*A,B | 312.9 (37.5)*A,B |
| | | | |
| 7a-OHase (pm/mg/min.) | 235.3(25.1) | 357.2(28.3)*A,B | 291.0(6.0)*A |
| 24 HR. FECAL Wt (G) | 2.3(0.1) | 2.7(0.1)*A,B | 2.4(0.04) |
| FBA(mM/24H/100g) | 6.2(0.8) | 12.3(1.5)*A,B | 11.9 (0.5)*A,B |

**TABLE 5.**

| **EFFICACY OF COMPOUND NO. 25 IN RAT ALZET MINIPUMP MODEL** | | |
|---|---|---|
| **PARAMETER** | **CONTROL** | **20 MPK/DAY CPD. NO. 25** |
| WEIGHT (G) | *(mean ± SEM, *p<0.05, A-Student's t, B.Dunnett's)* | |
| day 1 | 307(4) | 307(3) |
| day 8 | 330(4) | 310(4)*A,6 |
| LIVER WEIGHT (G) | 15.5(0.6) | 14.6(0.4) |
| SER.CHOL(mg%) | 85(3) | 84(3) |
| HEPATIC CHOL(mg/g) | 2.1(0.03) | 2.0(0.03) |
| HMG COA pm/mg/min | 75.1(6.4) | 318.0(40.7)*A,B |
| | | |
| 7a-OHase (pm/mg/min.) | 281.9(13.9) | 535.2(35.7)*A,B |
| 24 HR. FECAL Wt (G) | 5.8(0.1) | 5.7(0.4) |
| FBA(mM/24H/100g) | 17.9(0.9) | 39.1(4.5)*A,B |

The foregoing is merely illustrative of the invention and is not intended to limit the invention to the disclosed compounds. Variations and changes which are obvious to one skilled in the art are intended to be with in the scope and nature of the invention which are defined in the appended claims.

## Claims

1. A compound of the formula (I) 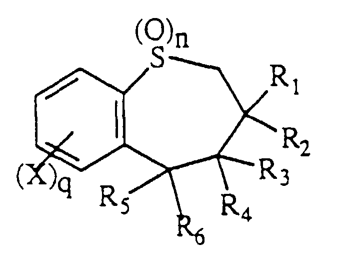 wherein
q is an integer of from 1 to 4;
n is independently an integer of from 0 to 2.
R₁ and R₂ are independently H, C₁-C₁₀-alkyl or R₁ and R₂ together form C₃-C₁₀-cycloalkyl;
R₃ and R₄ are independently H, C₁-C₆-alkyl, aryl, OR, NRR', S(O)ₙR, or R₃ and R₄ together form =0, =NOH, =S, =NNRR', =NR", =CRR' where R, R' and R" are selected from H, C₁-C₆-alkyl, C₂-C₆-alkenyl-C₁-C₆-alkyl, C₂-C₆-alkynyl-C₁-C₆-alkyl, aryl, carboxy-C₁-C₆-alkyl, carbo-C₁-C₆-alkoxy-C₁-C₆-alkyl, C₃-C₁₀-cycloalkyl, or cyan-C₁-C₆-alkyl; and provided that both R₃ and R₄ cannot be OH, NH₂ and SH;
R₅ is selected from C₁-C₆-alkyl, aryl, heterocycle, OR, NRR', S(O)ₙR wherein the C₁-C₆-alkyl, aryl, and heterocycle are each optionally substituted with C₁-C₆-alkyl, C₂-C₆-alkenyl, C₂-C₆-alkynyl, halogen, OR, NRR', S(O)ₙR, N0₂, halo-C₁-C₆-alkyl, carboxy, carbo-C₁-C₆-alkoxy, CN, or N+RR'R"Y- wherein R, R' and R" are each independently as defined above, and Y is independently an anion with the proviso that R₅ cannot be OH, NH₂, NRR' when R₁, R₂, R₃, R₄, and R₆ are all hydrogen or R and R' are hydrogen or C₁-C₆-alkyl; with the further proviso that R₅ cannot be OH or heterocyclic when R₁ and R₂ are hydrogen or C₁-C₆-alkyl and that R₅ cannot be C₁-C₆-alkyl or unsubstituted phenyl when R₁ and R₂ are both hydrogen and n is 0;
and that R₃, R₅ cannot both be thioaryl optionally substituted with C₁-C₆-alkyl, when R₄ is hydrogen and n is 0;
R₆ is selected from hydrogen or R₄ and R₆ together form -O-, or R₅ and R₆ together form a C₃-C₁₀-cycloalkylidene; with the proviso that R₄ and R₆ together cannot be -O- when R₃ is OH, NH₂ or SH;
X is selected from H, C₁-C₆-alkyl, C₂-C₆-alkenyl, C₂-C₆-alkynyl, halogen, OR, NRR', NROR', S(O)ₙR, N0₂, halo-C₁-C₆-alkyl, carboxy, carbo-C₁-C₆-alkoxy, CN, or N+RR'R"Y wherein R, R' and R" are each independently defined as above and Y is independently an anion; wherein aryl means phenyl or naphthyl;
wherein heterocycle means 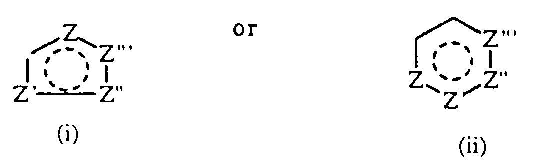 wherein Z, Z', Z" or Z"' is C, S, O, or N, with the proviso that one of Z, Z', Z" or Z"' is other than carbon, but is not O or S when attached to another Z atom by a double bond or when attched to another O or S atom, whereby optional substituents are attached to Z, Z', Z", Z"', only when each is C;
or pharmaceutically acceptable salt, or solvate thereof.

2. A compound of claim 1 wherein both R₁ and R₂ cannot both be hydrogen.

3. A compound of claim 1 wherein when R₅ is NRR', R₃ or R₄ cannot be aryl.

4. A compound of claim 1 wherein R₄ is OH and R₆ is hydrogen.

5. A compound of claim 1 wherein n is 2.

6. A compound of claim 1 wherein R₅ is aryl optionally substituted with C₁-C₆-alkyl, C₂-C₆-alkenyl, C₂-C₆-alkynyl, halogen, OR, NRR', S(O)ₙR, NO₂, halo-C₁-C₆-alkyl, carboxy, carbo-C₁-C₆-alkoxy, CN, or N+RR'R"Y.

7. A compound of claim 1 wherein R₃ and R₄ are independently H or OH.

8. A compound of claim 2 wherein R₄ and R₅ are in the same plane.

9. A compound of claim 3 wherein the compound is (3α,4β,5β) 3-Butyl-3-ethyl-4-hydroxy-5-phenyl-2,3,4,5-tetrahydrobenzothiepine-1,1-dioxide.

10. A compound of claim 1 wherein the compound is (3α,4β,5β) 3-Butyl-3-ethyl-4-hydroxy-8-methoxy-5-phenyl-2,3,4,5-tetrahydrobenzothiepine-1,1-dioxide.

11. A compound of claim 1 wherein the compound is (3α,4β,5β) 3-Butyl-3-ethyl-4-hydroxy-7-methoxy-5-phenyl-2,3,4,5-tetrahydrobenzothiepine-1,1-dioxide.

12. A compound of claim 1 wherein the compound is (3α,4β,5β) 3-Butyl-3-ethyl-4-hydroxy-7-hydroxyamino-5-phenyl-2,3,4,5-tetrahydrobenzothiepine-1,1-dioxide.

13. A compound of claim 1 wherein the compound is (3α,4β,5β) 3-Butyl-3-ethyl-4-hydroxy-7-amino-5-phenyl-2,3,4,5-tetrahydrobenzothiepine-1,1-dioxide.

14. A pharmaceutical composition for the prophylaxis or treatment of hyperlipidemic conditions wherein the condition is atherosclerosis which comprises an anti-atherosclerotic amount of a compound according to any of claims 1 - 13 with a pharmaceutically acceptable carrier.

15. A pharmaceutical composition for the prophylaxis or treatment of a hyperlipidemic condition wherein the condition is hypercholesterolemia which comprises an antihypercholesterolemia effective amount of a compound according to any of claims 1 - 13 together with a pharmaceutically acceptable carrier.

16. Use of a compound of Claim 12 in unit dosage form for preparing a medicament for the prophylaxis or treatment of hyperlipidemic conditions.

17. Use of a compound of Claim 13 in unit dosage form for preparing a medicament for the prophylaxis or treatment of atherosclerosis.

18. Use of a compound accoring to any of claims 1 - 13 including those wherein R₅ is OH or heterocyclic when R₁, R₂ are hydrogen or C₁-C₆-alkyl; and wherein R₅ is C₁-C₆-alkyl or unsubstituted phenyl when R₁, R₂ are both hydrogen and n is 0; and wherein R₃, R₅ are both arylthio optionally substituted with C₁-C₄-alkyl, when R₄ is hydrogen and n is 0; in unit dosage form for preparing a medicament for the prophylaxis or treatment of hypercholesterolemia or atherosclerosis.

## Patentansprüche

1. Eine Verbindung der Formel (I) 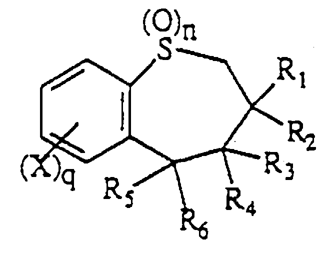 worin
q eine ganze Zahl von 1 bis 4 ist;
n unabhängig voneinander eine ganze Zahl von 0 bis 2 ist,
R₁ und R₂ unabhängig voneinander H, C₁-C₆-Alkyl sind oder R₁ und R₂ zusammen C₃-C₁₀-Cycloalkyl bilden;
R₃ und R₄ unabhängig voneinander H, C₁-C₆-Alkyl, Aryl, OR, NRR', S(O)ₙR sind oder R₃ und R₄ zusammen =O, =NOH, =S, =NNRR', =NR", =CRR' bilden, worin R, R' und R" ausgewählt sind aus H, C₁-C₆-Alkyl, C₂-C₆-Alkenyl-C₁-C₆-alkyl, C₂-C₆-Alkinyl-C₁-C₆-alkyl, Aryl, Carboxy-C₁-C₆-alkyl, Carbo-C₁-C₆-alkoxy-C₁-C₆-alkyl, C₃-C₁₀-Cycloalkyl oder Cyan-C₁-C₆-alkyl und mit der Maßgabe, daß beide R₃ und R₄ nicht OH, NH₂ und SH sein können;
R₅ ausgewählt ist aus C₁-C₆-Alkyl, Aryl, Heterocyclen, OR, NRR', S(O)ₙR, worin das C₁-C₆-Alkyl, Aryl und die Heterocyclen jeweils gegebenenfalls substituiert sind mit C₁-C₆-Alkyl, C₂-C₆-Alkenyl, C₂-C₆-Alkinyl, Halogen, OR, NRR', S(O)ₙR, NO₂ Halo-C₁-C₆-alkyl, Carboxy, Carbo-C₁-C₆-alkoxy, CN oder N+RR'R"Y- worin R, R' und R" jeweils unabhängig voneinander vorstehende Bedeutung haben und Y unabhängig voneinander ein Anion ist mit der Maßgabe, daß R₅ nicht OH, NH₂, NRR' sein kann, wenn R₁, R₂, R₃, R₄ und R₆ alle Wasserstoff sind oder R und R' Wasserstoff oder C₁-C₆-Alkyl sind; mit der weiteren Maßgabe, daß R₅ nicht OH oder heterocyclisch sein kann, wenn R₁ und R₂ Wasserstoff oder C₁-C₆-Alkyl sind und daß R₅ nicht C₁-C₆-Alkyl oder nichtsubstituiertes Phenyl sein kann, wenn R₁ und R₂ beide Wasserstoff sind und n 0 ist;
und daß R₃, R₅ nicht beide Thioaryl sein können, gegebenenfalls substituiert mit C₁-C₆-Alkyl, wenn R₄ Wasserstoff ist und n 0 ist;
R₆ ausgewählt ist aus Wasserstoff oder R₄ und R₆ zusammen -O- bilden, oder R₅ und R₆ zusammen ein C₃-C₁₀-Cycloalkyliden bilden; mit der Maßgabe, daß R₄ und R₅ zusammen nicht -O- sein können, wenn R₃ OH, NH₂ oder SH ist;
X ausgewählt ist aus H, C₁-C₆-Alkyl, C₂-C₆-Alkenyl, C₂-C₆-Alkinyl, Halogen, OR, NRR', NROR', S(O)ₙR, NO₂, Halo-C₁-C₆-alkyl, Carboxy, Carbo-C₁-C₆-alkoxy, CN oder N+RR'R"Y, worin R, R' und R" jeweils unabhängig voneinander vorstehende Bedeutung haben und Y unabhängig voneinander ein Anion ist, worin Aryl Phenyl oder Naphthyl bedeutet;
worin Heterocyclen 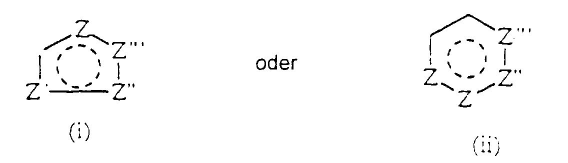 bedeuten, worin Z, Z', R" oder Z"' C, S, O oder N bedeuten, mit der Maßgabe, daß Z, Z', Z" oder Z"' etwas anderes als Kohlenstoff ist, jedoch nicht O oder S, wenn gebunden an ein anderes Z-Atom durch eine Doppelbindung oder wenn gebunden an ein anderes O- oder S-Atom, wobei gegebene Substituenten an Z, Z', Z", Z"' nur gebunden sind, wenn jedes C ist; oder pharmazeutisch verträgliche Salze oder Solvate davon.

2. Eine Verbindung des Anspruchs 1, worin R₁ und R₂ nicht beide Wasserstoff sein können.

3. Eine Verbindung des Anspruchs 1, worin, wenn R₅ NRR' ist, R₃ oder R₄ nicht Aryl sein können.

4. Eine Verbindung des Anspruchs 1, worin R₄ OH und R₆ Wasserstoff ist.

5. Eine Verbindung des Anspruchs 1, worin n 2 ist.

6. Eine Verbindung des Anspruchs 1, worin R₅ ein Aryl ist, das gegebenenfalls substituiert ist mit C₁-C₆-Alkyl, C₂-C₆-Alkenyl, C₂-C₆-Alkinyl, Halogen, OR, NRR', S(O)ₙR, NO₂, Halo-C₁-C₆-alkyl, Carboxy, Carbo-C₁-C₆-Alkoxy, CN oder N+RR'R"Y.

7. Eine Verbindung des Anspruchs 1, worin R₃ und R₄ unabhängig voneinander H oder OH sind.

8. Eine Verbindung des Anspruchs 2, worin R₄ und R₅ in der gleichen Ebene liegen.

9. Eine Verbindung des Anspruchs 3, worin die Verbindung (3α,4β,5β-)3-Butyl-3-ethyl-4-hydroxy-5-phenyl-2,3,4,5-tetrahydrobenzothiepin-1,1-dioxid ist.

10. Eine Verbindung des Anspruchs 1, worin die Verbindung (3α,4β,5β)-3-Butyl-3-ethyl-4-hydroxy-8-methoxy-5-phenyl-2,3,4,5-tetrahydrobenzothiepin-1,1-dioxid ist.

11. Eine Verbindung des Anspruchs 1, worin die Verbindung (3α,4β,5β)-3-Butyl-3-ethyl-4-hydroxy-7-methoxy-5-phenyl-2,3,4,5-tetrahydrobenzothiepin-1,1-dioxid ist.

12. Eine Verbindung des Anspruchs 1, worin die Verbindung (3α,4β,5β)-3-Butyl-3-ethyl-4-hydroxy-7-hydroxyamino-5-phenyl-2,3,4,5-tetrahydrobenzothiepin-1,1-dioxid ist.

13. Eine Verbindung des Anspruchs 1, worin die Verbindung (3α,4β,5β)-3-Butyl-3-ethyl-4-hydroxy-7-amino-5-phenyl-2,3,4,5-tetrahydrobenzothiepin-1,1-dioxid ist.

14. Eine pharmazeutische Zusammensetzung zur Prophylaxe oder Behandlung von hyperlipidämischen Zuständen, worin der Zustand Atherosklerose ist, enthaltend eine anti-atherosklerotische Menge einer Verbindung gemäß einem der Ansprüche 1-13 mit einem pharmazeutisch verträglichen Träger.

15. Eine pharmazeutische Zusammensetzung zur Prophylaxe oder Behandlung eines hyperlipidämischen Zustands, worin der Zustand Hypercholesterinämie ist, enthaltend eine gegen Hypercholesterinämie wirksame Menge einer Verbindung gemäß einem der Ansprüche 1-13 zusammen mit einem pharmazeutisch verträglichen Träger.

16. Verwendung einer Verbindung des Anspruchs 12 in Dosierungseinheitsform zur Herstellung eines Medikamentes zur Prophylaxe oder Behandlung von hyperlipidämischen Zuständen.

17. Verwendung einer Verbindung des Anspruchs 13 in Dosierungseinheitsform zur Herstellung eines Medikamentes zur Prophylaxe oder Behandlung von Atherosklerose.

18. Verwendung einer Verbindung gemäß einem der Ansprüche 1-13 einschließlich solcher, worin R₅ OH oder heterocyclisch ist, wenn R₁, R₂ Wasserstoff oder C₁-C₆-Alkyl sind und worin R₅ C₁-C₆-Alkyl oder nichtsubstituiertes Phenyl ist, wenn R₁, R₂ beide Wasserstoff sind und n 0 ist und worin R₃, R₅ beide Arylthio sind, gegebenenfalls substituiert mit C₁-C₄-Alkyl, wenn R₄ Wasserstoff ist und n 0 ist, in Dosierungseinheitsform zur Herstellung eines Medikamentes zur Prophylaxe oder Behandlung von Hypercholesterinämie oder Atherosklerose.

## Revendications

1. Composé de formule (I) 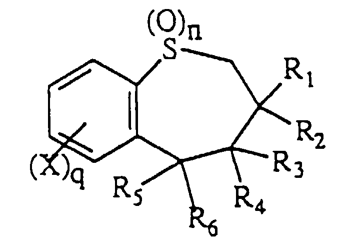 dans laquelle
q est un entier de 1 à 4;
n est indépendamment un entier de 0 à 2;
R₁ et R₂ sont indépendamment H ou un groupe alkyle en C₁-C₆, ou R₁ et R₂ forment ensemble un groupe cycloalkyle en C₃-C₁₀;
R₃ et R₄ sont indépendamment H ou un groupe alkyle en C₁-C₆, aryle, OR, NRR' ou S(O)ₙR, ou R₃ et R₄ forment ensemble un groupe =O, =NOH, =S, =NNRR', =NR" ou =CRR', dans lesquels R, R' et R" sont choisis parmi H et les groupes alkyle en C₁-C₆, (alcényl en C₂-C₆)-alkyle en C₁-C₆, (alcynyle en C₂-C₆)-alkyle en C₁-C₆, aryle, carboxy(alkyle en C₁-C₆), carbo(alcoxy en C₁-C₆)-alkyle en C₁-C₆, cycloalkyle en C₃-C₁₀ ou cyano(alkyle en C₁-C₆); à condition que R₃ et R₄ ne puissent pas être tous les deux un groupe OH, NH₂ et SH;
R₅ est choisi parmi les groupes alkyle en C₁-C₆, aryle, hétérocycliques, OR, NRR' ou S(O)ₙR, les groupes alkyle en C₁-C₆, aryle et hétérocycliques étant éventuellement substitués chacun par alkyle en C₁-C₆, alcényle en C₂-C₆, alcynyle en C₂-C₆, halogène, OR, NRR', S(O)ₙR, NO₂, halogénoalkyle en C₁-C₆, carboxy, carbo(alcoxy en C₁-C₆), CN ou N+RR'R"Y- où R, R' et R" ont chacun indépendamment la définition donnée ci-dessus, et Y est indépendamment un anion, à condition que R₅ ne puisse pas être un groupe OH, NH₂ ou NRR' lorsque R₁, R₂, R₃, R₄ et R₆ représentent tous l'hydrogène ou R et R' représentent l'hydrogène ou un groupe alkyle en C₁-C₆, à condition encore que R₅ ne puisse pas être un groupe OH ou hétérocyclique lorsque R₁ et R₂ représentent l'hydrogène ou un groupe alkyle en C₁-C₆, et que R₅ ne puisse pas être un groupe alkyle en C₁-C₆ ou phényle non substitué lorsque R₁ et R₂ représentent tous les deux l'hydrogène et que n est 0; et que R₃ et R₅ ne puissent pas représenter tous les deux un groupe thioaryle éventuellement substitué par alkyle en C₁-C₆ lorsque R₄ représente l'hydrogène et que n est 0;
R₆ représente l'hydrogène ou R₄ et R₆ forment ensemble -O-, ou R₅ et R₆ forment ensemble un groupe cycloalkylidène en C₃-C₁₀; à condition que R₄ et R₆ ne puissent pas être ensemble -O- lorsque R₃ est un groupe OH, NH₂ ou SH;
X est choisi parmi H et les groupes alkyle en C₁-C₆, alcényle en C₂-C₆, alcynyle en C₂-C₆, halogène, OR, NRR', NROR', S(O)ₙR, NO₂, halogénoalkyle en C₁-C₆, carboxy, carbo(alcoxy en C₁-C₆), CN ou N+RR'R"Y où R, R' et R" ont chacun indépendamment la définition donnée ci-dessus, et Y est indépendamment un anion;
les groupes aryle désignant des groupes phényle ou naphtyle;
les groupes hétérocycliques désignant les groupes 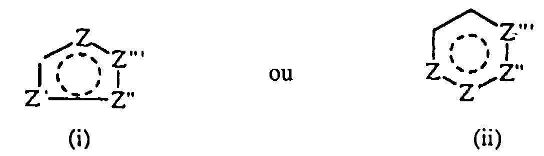 dans lesquels Z, Z', Z" ou Z"' est C, S, O ou N, à condition que l'un des Z, Z', Z" ou Z"' soit différent du carbone, mais ne soit ni O, ni S lorsqu'il est lié à un autre atome Z par une double liaison ou lorsqu'il est lié à un autre atome O ou S, des substituants éventuels étant liés à Z, Z', Z", Z"' uniquement lorsqu'ils sont chacun C;
ou un de ses sels pharmaceutiquement acceptables ou un de ses solvates.

2. Composé selon la revendication 1, dans lequel R₁ et R₂ ne peuvent pas être tous les deux l'hydrogène.

3. Composé selon la revendication 1, dans lequel, lorsque R₅ est un groupe NRR', R₃ ou R₄ ne peut pas être un groupe aryle.

4. Composé selon la revendication 1, dans lequel R₄ est OH et R₆ est l'hydrogène.

5. Composé selon la revendication 1, dans lequel n est 2.

6. Composé selon la revendication 1, dans lequel R₅ est un groupe aryle éventuellement substitué par alkyle en C₁-C₆, alcényle en C₂-C₆, alcynyle en C₂-C₆, halogène, OR, NRR', S(O)ₙR, NO₂, halogénoalkyle en C₁-C₆, carboxy, carbo(alcoxy en C₁-C₆), CN ou N⁺RR'R"Y⁻.

7. Composé selon la revendication 1, dans lequel R₃ et R₄ sont indépendamment H ou OH.

8. Composé selon la revendication 2, dans lequel R₄ et R₅ sont dans le même plan.

9. Composé selon la revendication 3, ce composé étant le (3α,4β,5β)-3-butyl-3-éthyl-4-hydroxy-5-phényl-2,3,4,5-tétrahydrobenzothiépine-1,1-dioxyde.

10. Composé selon la revendication 1, ce composé étant le (3α,4β,5β)-3-butyl-3-éthyl-4-hydroxy-8-méthoxy-5-phényl-2,3,4,5-tétrahydrobenzothiépine-1,1-dioxyde.

11. Composé selon la revendication 1, ce composé étant le (3α,4β,5β)-3-butyl-3-éthyl-4-hydroxy-7-méthoxy-5-phényl-2,3,4,5-tétrahydrobenzothiépine-1,1-dioxyde.

12. Composé selon la revendication 1, ce composé étant le (3α,4β,5β)-3-butyl-3-éthyl-4-hydroxy-7-hydroxyamino-5-phényl-2,3,4,5-tétrahydrobenzothiépine-1,1-dioxyde.

13. Composé selon la revendication 1, ce composé étant le (3α,4β,5β)-3-butyl-3-éthyl-4-hydroxy-7-amino-5-phényl-2,3,4,5-tétrahydrobenzothiépine-1,1-dioxyde.

14. Composition pharmaceutique pour la prophylaxie ou le traitement d'un état hyperlipidémique, cet état étant l'athérosclérose, qui comprend une quantité antiathéroscléreuse d'un composé selon l'une quelconque des revendications 1 à 13 avec un support pharmaceutiquement acceptable.

15. Composition pharmaceutique pour la prophylaxie ou le traitement d'un état hyperlipidémique, cet état étant l'hypercholestérolémie, qui comprend une quantité efficace anti-hypercholestérolémique d'un composé selon l'une quelconque des revendications 1 à 13 avec un support pharmaceutiquement acceptable.

16. Utilisation d'un composé selon la revendication 12 sous forme galénique unitaire pour la préparation d'un médicament destiné à la prophylaxie ou au traitement d'états hyperlipidémiques.

17. Utilisation d'un composé selon la revendication 13 sous forme galénique unitaire pour la préparation d'un médicament destiné à la prophylaxie ou au traitement de l'athérosclérose.

18. Utilisation d'un composé selon l'une quelconque des revendications 1 à 13, y compris de ceux dans lesquels R₅ est OH ou un groupe hétérocyclique lorsque R₁ et R₂ sont l'hydrogène ou des groupes alkyle en C₁-C₆, et dans lesquels R₅ est un groupe alkyle en C₁-C₆ ou phényle non substitué lorsque R₁ et R₂ sont tous les deux l'hydrogène et n est 0, et dans lesquels R₃ et R₅ sont tous les deux un groupe arylthio éventuellement substitué par alkyle en C₁-C₄ lorsque R₄ est l'hydrogène et n est 0, sous forme galénique unitaire pour la préparation d'un médicament destiné à la prophylaxie ou au traitement de l'hypercholestérolémie ou de l'athérosclérose.
